# EUROPÄISCHE PATENTSCHRIFT

(11) **EP 3 236 775 B1**
(45) Veröffentlichungstag und Bekanntmachung des Hinweises auf die Patenterteilung: **28.08.2019**
(21) Anmeldenummer: 15832790.8
(22) Anmeldetag: 22.12.2015
(51) Int. Cl.: A23L 2/46, A23L 3/02, A23L 3/18, A23L 3/28, A61L 9/00, A61L 2/10, C02F 9/00, B65B 55/02, A23L 3/00, A61L 2/02, C12H 1/16, C02F 1/28, C02F 1/02, C02F 1/32, C02F 1/44, C02F 103/02

(54) **VERFAHREN UND VORRICHTUNG ZUR BEHANDLUNG VON LEBENSMITTELN IN GESCHLOSSENEN BEHÄLTNISSEN MITTELS EINER PROZESSFLÜSSIGKEIT**
METHOD AND DEVICE FOR TREATING FOOD IN CLOSED CONTAINERS BY MEANS OF A PROCESS LIQUID
PROCÉDÉ ET INSTALLATION DE TRAITEMENT DE PRODUITS ALIMENTAIRES DANS DES RÉCIPIENTS FERMÉS AU MOYEN D'UN FLUIDE DE TRAITEMENT

(30) Priorität: 22.12.2014 AT 509352014; 22.07.2015 AT 506462015
(43) Veröffentlichungstag der Anmeldung: 01.11.2017
(73) Patentinhaber: Red Bull GmbH, 5330 Fuschl am See (AT)
(72) Erfinder: CONCIN, Roland, 5330 Fuschl am See (AT); EDER, Harald, 5301 Eugendorf (AT); RINDERER, Christian, 5330 Fuschl am See (AT); RINDERER, Matthias, 5330 Fuschl am See (AT); VIECHTBAUER, Volker, 5330 Fuschl am See (AT)
(74) Vertreter: Burger, Hannes
(86) Internationale Anmeldenummer: PCT/AT2015/050327
(87) Internationale Veröffentlichungsnummer: WO 2016/100997

(56) Entgegenhaltungen:
- DE-U1- 29 616 876
- DE-U1- 29 616 876
- JP-A- 2014 128 802
- JP-B2- 3 922 935
- JP-B2- 3 922 935
- US-A1- 2003 205 514
- US-A1- 2003 205 514
- US-A1- 2012 070 540
- US-A1- 2012 070 540
- FAEHNRICH M ET AL: "AUFBEREITUNG VON SCHWACH BELASTETEN PROZESSABWAESSERN IN DER LEBENSMITTELINDUSTRIE", CHEMIE INGENIEUR TECHNIK, WILEY VCH. VERLAG, WEINHEIM; DE, Bd. 69, Nr. 8, 1. August 1997 (1997-08-01) , XP000829070, ISSN: 0009-286X, DOI: 10.1002/CITE.330690819
- FAEHNRICH M ET AL: "AUFBEREITUNG VON SCHWACH BELASTETEN PROZESSABWAESSERN IN DER LEBENSMITTELINDUSTRIE", CHEMIE INGENIEUR TECHNIK, WILEY VCH. VERLAG, WEINHEIM; DE, vol. 69, no. 8, 1 August 1997 (1997-08-01) , page 1147/1148, XP000829070, ISSN: 0009-286X, DOI: 10.1002/CITE.330690819

## Beschreibung

Die Erfindung betrifft ein Verfahren zur Behandlung von Lebensmitteln und/oder Behältnissen zur Aufnahme von Lebensmitteln, sowie eine Vorrichtung für die Behandlung von Lebensmitteln und/oder Behältnissen zur Aufnahme von Lebensmitteln. Im Besonderen betrifft die Erfindung ein Verfahren und eine Vorrichtung zur Behandlung von Genussmitteln, insbesondere alkoholischen und nichtalkoholischen Getränken. Die Lebensmittel bzw. die Behältnisse zur Aufnahme der Lebensmittel, werden dabei mittels einer Prozessflüssigkeit in zumindest einer Behandlungszone behandelt, wobei die Prozessflüssigkeit nach Abführung aus der oder den Behandlungszone(n) zur Wiederverwendung im Verfahren im Kreis der oder den Behandlungszone(n) wieder zugeführt wird. Zur Reinigung und Entkeimung wird eine Teilmenge der im Kreis geführten Prozessflüssigkeit zur Bildung zumindest eines Stromes der Prozessflüssigkeit herangezogen, und der wenigstens eine gebildete Strom mittels zumindest einer Membranfiltrationsanlage filtriert und/oder mittels zumindest einer UV-Bestrahlungseinrichtung bestrahlt.

Verfahren bzw. Vorrichtungen zur Behandlung von Lebensmitteln und/oder Behältnissen sind in verschiedenen Varianten aus dem Stand der Technik bekannt. Häufig werden die Produkte, zum Beispiel Lebensmittel, und/oder die Behältnisse dabei mittels einer temperierten Prozessflüssigkeit behandelt, wie dies zum Beispiel bei Pasteurisierungen von Lebensmittelprodukten in sogenannten Pasteuren der Fall ist. In den meisten Fällen wird als Prozessflüssigkeit Wasser bzw. eine wässrige Lösung eingesetzt, welche indirekt auf die Produkte bzw. direkt auf die Behältnisse einwirkt.

Zwecks Vermeidung großer Mengen an Flüssigkeitsabfall bzw. Abwasser wird die Prozessflüssigkeit bzw. das Prozesswasser häufig zumindest teilweise im Kreis durch die Vorrichtung bzw. die Behandlungszone(n) geführt, die Prozessflüssigkeit wird also in einem Umlaufverfahren wiederverwendet. Diese Vorgangsweise bedingt allerdings ein erhöhtes Potential für eine Verschmutzung bzw. Kontamination der Prozessflüssigkeit, insbesondere eine erhöhte Gefahr des Wachstums von Keimen bzw. pathogenen Mikroorganismen. Anlagen zur Behandlung von Produkten bzw. Behältnissen sind in den meisten Fällen begehbar, es sind also keineswegs Bedingungen mit kontrollierter Luftumwälzung oder dergleichen gegeben. Zusätzlich zur Umgebungsluft sind weitere Schmutz- und Keimquellen, wie zum Beispiel das Bedienungspersonal oder andere Personen, Förderelemente für die Behältnisse bzw. die Produkte, etwaig vorhandene Kühleinrichtungen für die Prozessflüssigkeit, insbesondere luftgekühlte Kühltürme, oder aber die frisch in das Verfahren bzw. die Vorrichtung eingebrachte Prozessflüssigkeit selbst, vorhanden. Weiters kann ein Eintrag von Verunreinigungen durch den Behandlungsprozess selbst erfolgen, etwa durch Beschädigungen der Behältnisse und Verunreinigung der Prozessflüssigkeit durch das Produkt, oder durch Ablösen von Partikeln, beispielsweise Farbpartikeln oder dergleichen, von der Außenseite der Behältnisse.

In der Vergangenheit wurden einige Verfahren vorgeschlagen, um Verunreinigungen aus einer Prozessflüssigkeit zu entfernen. Es handelt sich hierbei um Filtrationsmaßnahmen zur Entfernung verhältnismäßig grober bzw. großer Partikel, wie etwa Glasscherben, Sand, Kies und dergleichen. Als Beispiel sei die EP 2 722 089 A1 genannt. In der EP 2 722 089 A1 ist eine Vorrichtung zur thermischen Behandlung von Produkten in Behältern beschrieben. Die Behälter werden dabei mit einer Prozessflüssigkeit berieselt bzw. besprüht, und die Prozessflüssigkeit, beispielsweise Wasser, wird zum zumindest teilweisen Wiederverwenden in einem Umlaufkreislauf geführt. Zur Reinigung der Prozessflüssigkeit kommt eine Schwerkraftsedimentationsvorrichtung zum Einsatz, mittels welcher grobkörnige bzw. große Partikel, wie Glasscherben oder Sand abgeschieden werden können.

Die aus dem Stand der Technik bekannten Methoden beschreiben beispielsweise Filtrationsmethoden zur Abscheidung von verhältnismäßig großen Partikeln aus einer Prozessflüssigkeit, mittels Abscheidungsvorrichtungen wie zum Beispiel konventionellen Maschensieben, Siebbändern oder Stecksieben, oder eben Sedimentationsvorrichtungen.

Diese aus dem Stand der Technik bekannten Maßnahmen sind jedoch nicht geeignet, um einerseits Verschmutzungen mit verhältnismäßig kleiner Partikelgröße aus der Prozessflüssigkeit zu entfernen. Dies betrifft besonders Partikelverunreinigungen, welche durch konventionelle Filtersiebe und sonstige Abscheidevorrichtungen nicht entfernt werden können, bzw. sich zum Beispiel nicht oder nur sehr langsam aus einem Prozesswasserstrom absetzen. Des Weiteren können Mikroorganismen bzw. pathogene Keime so nicht in ausreichendem Maße aus der Prozessflüssigkeit bzw. dem Prozesswasser entfernt werden. Insbesondere stellen diese vorbekannten Maßnahmen kein wirksames Mittel dar, um eine Vermehrung bzw. ein Wachstum von Mikroorganismen bzw. Keimkolonien zu unterbinden bzw. zumindest zu verringern. Hierzu ist der weitreichende Einsatz keimdeaktivierend wirkender Chemikalien wie etwa Chlor oder Chlorierungsmittel nötig, welche Chemikalien größtenteils gesundheits- und umweltschädlich sein können. Außerdem ist der Einsatz von Chemikalien zur Desinfektion in der Regel mit hohen Kosten verbunden.

Bei derartigen Vorrichtungen bzw. Anlagen zur Behandlung von Produkten bzw. Lebensmitteln sind darüber hinaus zumindest in Teilbereichen Bedingungen gegeben, welche ein Wachstum bzw. eine Vermehrung von Mikroorganismen in der Prozessflüssigkeit bzw. dem Prozesswasser begünstigen, beispielsweise aufgrund einer besonders geeigneten, wachstumsfördernden Temperatur. Diese Mikroorganismen, zum Beispiel Bakterien, Pilzsporen, aber auch Viren, können dabei sowohl mit frischem Prozesswasser, oder aber auch durch Bedienungspersonal oder andere Personen in die Vorrichtung eingebracht werden. Grundsätzlich kann dabei nicht gänzlich ausgeschlossen werden, dass Mikroorganismen im Zuge der Behandlung auf die Außenseite der Behälter gelangen, und dort zumindest teilweise auch nach dem Behandlungsvorgang verbleiben.

Als besonders problematisch ist die hinsichtlich einer Filtration mittels konventionellen Sieben ungünstige Partikelgrößenverteilung in einem typischen Prozesswasser anzusehen. Insbesondere begünstigt der Einsatz von Chemikalien wie etwa Tensiden sehr kleine Partikelgrößen, welche mit konventionellen Abscheidevorrichtungen nicht oder nur in unzureichendem Ausmaß aus der Prozessflüssigkeit entfernt werden können. Letztlich muss der Produktions- bzw. Behandlungsbetrieb in derzeit bekannten Verfahren zur Behandlung von Produkten bzw. Behältnissen, in welchen die Prozessflüssigkeit zumindest teilweise im Kreis geführt wird, in verhältnismäßig kurzen Intervallen unterbrochen werden, um die Behandlungsvorrichtung zu reinigen und zu entkeimen. Solche Reinigungs- und Entkeimungsvorgänge sind in der Regel sehr aufwendig, und führen insbesondere zu Produktionsverlusten, und damit einhergehend zu finanziellen Verlusten.

Aus dem Stand der Technik sind grundsätzlich Reinigungs- bzw. Wasseraufbereitungsmethoden bekannt, welche Membranfiltrationstechniken und UV-Bestrahlung in Kombination nut zen. Zum Beispiel ist aus Faehnrich M et al:" Aufbereitung von schwach belasteten Prozessabwässern in der Lebensmittelindustrie", Chemie Ingenieur Technik, Wiley Vch. Verlag, Weinheim; DE, Bd. 69, Nr.8, 1. August 1997 bekannt, Abwasser, welches beim Spülen von Fleischprodukten mittels Kühlduschen anfällt zu sammeln, mittels Nanofiltration und UV-Bestrahlung aufzubereiten und wiederzuverwenden.

Die US 2003/205514 A1 offenbart ein Prozesswasser-Aufbereitungs- und Recyclingsystem für Lebensmittel verarbeitende Prozesse, bei welchen das Prozesswasser ebenfalls direkt mit den Lebensmitteln in Kontakt ist. Abwasser aus dem Prozess wird in Sammeltanks gesammelt, und dem Aufbereitungssystem zugeführt. Hier wird das gesammelte Prozesswasser mittels Edelstahlmembranen gefiltert, und optional mittels UV-Strahlung entkeimt. Das aufbereitete Prozesswasser kann für einen Frischwasserstrom für die Lebensmittel verarbeitenden Prozesse benutzt werden. Das Aufbereitungs- und Recyclingsystem erleichtert dabei die Entfernung von Nebenprodukten, wie etwa Stärke aus dem Prozesswasser, und die Wiederverwendung dieser Nebenprodukte.

Die US 2012/070540 A1 offenbart ein Herstellungsverfahren für Wasserstoffhaltiges Trinkwasser. Hierzu wird Rohwasser über Aktivkohle und einen Filter geführt, anschließend entgast und mit Wasserstoff versetzt. Vor dem Abfüllen sind noch eine UV-Bestrahlung und eine Membranfiltration vorgesehen. Die DE 296 16 876 U1 offenbart eine Vorrichtung zum Sterilisieren/Desinfizieren von medizinischen Materialien und Instrumenten, wobei eine Reinigungslösung vor dem Desinfizieren/Sterilisieren der medizinischen Geräte mittels Filter, etwa Aktivkohlefilter oder Membranfilter aufbereitet wird. Zur Vorbereitung der Reinigungslösung können auch UV-Strahler eingesetzt werden.

Aus der JP 3 922935 B2 ist ein Wasseraufbereitungssystem bekannt, welchem flüchtige, organische Substanzen aus einem Prozesswasser entfernt werden. Das Wasseraufbereitungssystem ist für Prozesswasser aus der Halbleiter- bzw. Elektronikproduktion, bzw. zur Herstellung von ultrareinem Wasser vorgesehen. Organische Substanzen werden durch Kombinationen aus Umkehrosmose und UV-Licht unterstütze, oxidative Zersetzung mit anschließender Verdampfung der flüchtigen, organischen Verbindungen in einem Verdampfungsvorgang durch eine hydrophobe Membran hindurch, aus dem Wasser entfernt.

Aufgabe der Erfindung ist es daher, ein verbessertes Verfahren und eine verbesserte Vorrichtung zu entwickeln, welche die im Stand der Technik noch vorhandenen Defizite zu beheben vermögen. Insbesondere sollen ein im Vergleich zum Stand der Technik, verbessertes Verfahren bzw. eine verbesserte Vorrichtung zur Behandlung von Produkten, insbesondere Lebensmittelprodukten, und/oder Behältnissen bereitgestellt werden, bei welchem Verfahren bzw. bei welcher Vorrichtung die Prozessflüssigkeit zur Wiederverwendung zumindest teilweise im Verfahren im Kreis geführt werden kann, aber die damit einhergehenden Nachteile durch die kontinuierlich steigende Verunreinigung bzw. Verkeimung der Prozessflüssigkeit möglichst hintangehalten sind.

Die Aufgabe der Erfindung wird gemäss Anspruch 1 gelöst.

Die Lebensmittel in den Behältnissen werden dabei in eine Behandlungszone eingebracht bzw. durch eine Behandlungszone befördert. Der Behandlungszone oder den Behandlungszonen wird jeweils zumindest ein Flüssigkeitsstrom der Prozessflüssigkeit zum Einwirken auf die Lebensmittel bzw. die Behältnisse zugeführt, und nach erfolgter Behandlung der Lebensmittelprodukte in den Behältnissen aus der Behandlungszone wieder abgeführt, wobei die Prozessflüssigkeit zur Behandlung der Lebensmittel bzw. der Behältnisse zwecks Wiederverwendung im Verfahren im Kreis wieder in die Behandlungszone oder in die Behandlungszonen geführt wird.

Im kontinuierlichen, laufenden Behandlungsbetrieb wird, aus der pro Zeiteinheit insgesamt durch alle vorhandenen Behandlungszonen geführten Prozessflüssigkeit, pro Zeiteinheit eine Teilmenge der Prozessflüssigkeit zur Bildung wenigstens eines Stromes der Prozessflüssigkeit herangezogen, und der wenigstens eine gebildete Strom der Prozessflüssigkeit zur Reinigung und Entkeimung der Prozessflüssigkeit mittels zumindest einer Membranfiltrationsanlage filtriert und/oder mittels zumindest einer UV-Bestrahlungseinrichtung bestrahlt, und ein bestrahlter und/oder filtrierter Strom nach dem Filtrationsvorgang zumindest teilweise einem die Prozessflüssigkeit beinhaltenden bzw. führenden Element und/oder einer Behandlungszone wieder zugeführt.

Unter einem die Prozessflüssigkeit beinhaltenden bzw. führenden Element, bzw. Führungselement für die Prozessflüssigkeit wird hier und im Folgenden jegliches Element verstanden, welches zur Beinhaltung der Prozessflüssigkeit bzw. zur Leitung eines Flüssigkeitsstromes der Prozessflüssigkeit ausgestaltet ist. Das können beispielsweise Rohrleitungen, Kanäle und dergleichen sein, in welchen die Prozessflüssigkeit zum Beispiel einer Behandlungszone zugeführt oder aus einer Behandlungszone abgeführt wird. Weiters werden unter dem Begriff "Führungselement" beispielsweise, aber nicht ausschließend Sammelbecken, Tanks, oder in den oder außerhalb der Behandlungszonen angeordneten Auffangeinrichtungen für die Prozessflüssigkeit oder dergleichen verstanden.

Unter einer Behandlungszone wird hier und im Folgenden eine Zone verstanden, in welcher die Lebensmittel vorzugsweise indirekt und/oder die Behältnisse vorzugsweise direkt mit der Prozessflüssigkeit in Kontakt gebracht werden. Dabei können die physikalischen und/oder chemischen und/oder sonstigen Parameter der Prozessflüssigkeit gezielt für den jeweiligen Behandlungszweck der Zone eingestellt sein bzw. werden. Die Einwirkung der Prozessflüssigkeit auf die Lebensmittel bzw. die Behältnisse für die Aufnahme von Lebensmitteln kann auf verschiedene Art und Weise erfolgen. Beispielsweise können zur Generierung der gewünschten Wechselwirkung die Prozessflüssigkeit und ein zu behandelndes, flüssiges Lebensmittel stofflich voneinander getrennt im Gegenstrom- oder Gleichstrom- oder Kreuzstromprinzip in jeweils aneinander angrenzenden Führungselementen geleitet werden. Die gewünschte Wechselwirkung kann dabei durch Wärmeübertragung zwischen Lebensmittel und Prozessflüssigkeit in im Sinne eines Wärmetauschers erzielt werden, wie dies beispielsweise bei der Pasteurisierung zum Haltbarmachen von Milch üblich ist. Ein weiteres häufig angewandtes Verfahren ist die Pasteurisierung von Lebensmittelprodukten, bei welchem sich die Lebensmittel bereits in verschlossenen Behältnissen befinden, und die Prozessflüssigkeit auf die Außenseite der Behältnisse einwirkt. Dabei kann die Prozessflüssigkeit auf die Außenseite der Behältnisse geschüttet, bzw. die Behältnisse mit der Prozessflüssigkeit berieselt oder besprüht werden. Als weiteres Beispiel sind aber auch Tauchverfahren möglich, bei welchen die ein Lebensmittel beinhaltenden Behältnisse in die Prozessflüssigkeit getaucht werden. Selbstverständlich kann das erfindungsgemäße Verfahren bzw. die erfindungsgemäße Vorrichtung aber auch zur Behandlung, zum Beispiel zum Spülen/Reinigen von leeren Behältnissen angewandt werden. Letztlich ist unter dem Begriff Behandlungszone somit zumindest im weitesten Sinne auch ein die Prozessflüssigkeit beinhaltendes bzw. führendes Element, bzw. Führungselement für die Prozessflüssigkeit zu verstehen.

Unter einem Flüssigkeitsstrom der Prozessflüssigkeit wird hier und im Folgenden jegliche Art von geführt bewegter Prozessflüssigkeit verstanden, unabhängig davon wie der Flüssigkeitsstrom bzw. dessen Führung ausgestaltet sind. Das heißt, dass unter dem Begriff "Flüssigkeitsstrom" zum Beispiel ein Strom einer bewegten Prozessflüssigkeit in Führungselementen, wie etwa Rohrleitungen, Kanälen, Becken, Tanks etc. genauso umfasst ist, wie auch beispielsweise ein unter Umgebungsluftdruck frei fallender Berieselungsstrom oder Sprühstrom der Prozessflüssigkeit in einer Behandlungszone oder ein Strom der Prozessflüssigkeit in einer Rückkühleinrichtung oder dergleichen.

Durch die im Anspruch 1 angegebenen Maßnahmen wird ein Verfahren bereitgestellt, welches sich besonders für die Entfernung von Verschmutzungen bzw. Kontaminationen mit sehr kleiner Partikelgröße, wie etwa Bakterienkolonien aus der Prozessflüssigkeit, sowie zur Entkeimung der Prozessflüssigkeit hervorragend eignet. Damit lässt sich eine wesentliche Verbesserung gegenüber vorbekannten Verfahren erreichen, welche hinsichtlich einer Entfernung kleiner Partikel, sowie lebensfähiger bzw. vermehrungsfähiger Mikroorganismen aus einer zumindest teilweise im Kreis geführten Prozessflüssigkeit nur sehr eingeschränkt, oder gar nicht wirksam sind. Die Reinigung und Entkeimung der Prozessflüssigkeit kann dabei vorteilhafterweise im laufenden Behandlungsbetrieb durchgeführt werden, und ist vergleichsweise effizient und energiesparend.

Des Weiteren ist die Membranfiltration per se wirksam in der Entfernung von Mikroorganismen, wodurch sich in synergistischer Art und Weise durch Kombination einer Membranfiltration und einer UV-Bestrahlung die Effizienz einer Verminderung vermehrungsfähiger Mikroorganismen deutlich steigern lässt. Insbesondere bei Durchführung der Membranfiltration als sogenannte, Ultrafiltration' also Filtration mit Membranen mit Porendurchmessern von ca. 0,2 m oder weniger, können sowohl anorganische als auch organische Klein- und Kleinstpartikel, beispielsweise Bakterienkolonien, wirksam aus der Prozessflüssigkeit entfernt werden.

Die genaue Anzahl, sowie Art und Ort der Einbindung der Membranfiltrationsanlage(n) und UV-Bestrahlungseinrichtung(en) bzw. die Anknüpfung einer Membranfiltrationsanlage und einer UV-Bestrahlungseinrichtung an die die Prozessflüssigkeit leitenden Führungselemente und/oder Behandlungszone(n), kann dabei unter Berücksichtigung von baulichen Merkmalen bzw. Prozessparametern und dergleichen festgelegt bzw. vorgenommen werden. Jedoch können sich bei bestimmten Anordnungsvarianten Vorteile für das Verfahren zur Behandlung von Lebensmitteln bzw. Behältnissen ergeben, welche nachstehend noch näher erläutert werden.

Zur Entfernung großkörniger Verunreinigungen in der Prozessflüssigkeit können beispielsweise zusätzlich Schmutzfänger oder dergleichen in den Führungselementen angeordnet sein. Zum Beispiel kann auch eine Schwerkraftsedimentationsvorrichtung, wie sie in der bereits zitierten EP 2 722 089 A1 beschrieben ist, zur Abscheidung von großen bzw. großkörnigen Partikeln eingesetzt werden.

Durch die in Anspruch 1 angegebenen Maßnahmen wird des Weiteren erreicht, dass aufwendige und kostspielige Unterbrechungen des Produktions- bzw. Behandlungsbetriebs zwecks Reinigung der Behandlungsvorrichtung möglichst hintangehalten werden können, bzw. zumindest die Zeitintervalle zwischen solchen Reinigungsprozessen wesentlich vergrößert werden können. Zusätzlich kann durch die Mikro- bzw. Ultrafiltration und die UV-Bestrahlung zumindest die Menge an chemischen Stabilisatoren zur Aufbereitung der Prozessflüssigkeit, insbesondere die benötigten Mengen an Tensiden und Korrosionsinhibitoren, sowie von Desinfektionsmitteln bzw. Bioziden reduziert werden, bzw. kann der Einsatz solcher Reinigungschemikalien und Desinfektionschemikalien zumindest größtenteils eingespart bzw. minimiert werden. Dabei ist die Membranfiltration sowohl hinsichtlich Entfernung von Mikroorganismen als auch anderer Verunreinigungen wirksam. Durch die UV-Bestrahlung kann die Effizienz des Verfahrens hinsichtlich Entkeimung noch weiter gesteigert werden.Durch die Membranfiltration kann außerdem die Wirksamkeit bzw. Effizienz der UV-Bestrahlungseinrichtung(en) deutlich verbessert werden. Klein- und Kleinstpartikel sowie Schwebstoffe können eine Trübung der Prozessflüssigkeit verursachen, welche die Wirksamkeit der UV-Bestrahlung deutlich herabsetzen kann. Mittels einer Membranfiltrationsanlage kann die Trübung der Prozessflüssigkeit deutlich verringert werden, und damit Effizienzverluste der UV-Bestrahlung durch UV-Licht-Absorption, -Streuung etc. hintangehalten werden. Die Trübung einer Flüssigkeit kann beispielsweise durch die Einheit NTU (Nephelometrischer Trübungswert) angegeben werden. Bevorzugt weist die Prozessflüssigkeit eine Trübung von weniger als 5 NTU, insbesondere weniger als 1 NTU auf.

Klein- und Kleinstpartikel bzw. eine Trübung verursachende Stoffe können unter Anderem im Zuge der Herstellung der Behältnisse durch formgebende bzw. spanende Bearbeitungsschritte wie etwa Schneiden, Fräsen, Bohren oder dergleichen verursacht werden bzw. durch die Behältnisse in die Prozessflüssigkeit gelangen. Dabei wird bei typischen Vorrichtungen zur Behandlung von Lebensmitteln und/oder Behältnissen insbesondere die Bildung von Klein- und Kleinstpartikel aufgrund des Einsatzes von Chemikalien wie etwa Tensiden begünstigt.

In weiterer Folge kann eine wesentliche Verbesserung hinsichtlich Umweltschonung erzielt werden, bzw. auch die Belastung einer dem Verfahren bzw. der Vorrichtung nachgeschalteten Einrichtung, wie etwa einer Kläranlage, minimiert werden. Zusätzlich ist die Bestrahlung mit UV-Licht auch wirksam hinsichtlich einer Unterdrückung einer Vermehrung zumindest der meisten Algenarten.

Außerdem können durch die erfindungsgemäßen Maßnahmen auch olfaktorische Faktoren verbessert werden, da eine Bildung ungewünschter bzw. unangenehmer Gerüche hintangehalten werden kann. Diverse Mikroorganismen, insbesondere Bakterien sind bekannt dafür, übel riechende Duftstoffe als Stoffwechselprodukte zu generieren. Durch die Entfernung bzw. Abtötung dieser Mikroorganismen kann eine unangenehme Geruchsbelastung weitestgehend vermieden werden.

Bei längerem Verbleib von Bakterienkulturen in einer Prozessflüssigkeit unter gleichzeitigem Einsatz von Bioziden, können sich Bakterienstämme an die eingesetzten Biozide "gewöhnen" bzw. gegenüber den eingesetzten Bioziden resistente Bakterienstämme gebildet werden. Das heißt, dass Biozide zur Entfernung von Mikroorganismen aus der Prozessflüssigkeit mit der Zeit eingeschränkt wirksam oder sogar unwirksam werden können. Durch die kontinuierliche Entfernung der Mikroorganismen aus der Prozessflüssigkeit und die zusätzliche UV-Bestrahlung der Prozessflüssigkeit, kann die Bildung resistenter Keime hintangehalten werden, bzw. kann der Einsatz von Bioziden insgesamt minimiert werden, da effiziente und wirksame Mittel bereitgestellt sind, um das Wachstum bzw. die Vermehrung von Mikroorganismen in der Prozessflüssigkeit hintanzuhalten. Durch die Membranfiltration und UV-Bestrahlung kann beispielsweise auch das Wachstum biozidresistenter und/oder chlorresistenter Bakterienstämme wirksam hintangehalten werden.

Selbstverständlich können auch andere organische und anorganische Klein- und Kleinstpartikel mittels der zumindest einen Membranfiltrationsanlage aus der Prozessflüssigkeit entfernt werden. Dadurch ergibt sich auch gleichzeitig eine weitere Verbesserung hinsichtlich des Mikroorganismenwachstums bzw. hinsichtlich der Wachstumsrate von Mikroorganismen in der Prozessflüssigkeit, da organische und anorganische Klein- und Kleinstpartikel oft gute Nährgrundlagen bzw. "Nährböden", beispielsweise für Bakterien bilden können. Durch das Entfernen von unerwünschten Partikelverunreinigungen mittels der Membranfiltration, kann auch der Einsatz von sonst notwendigen Chemikalien zur Eliminierung solcher Verunreinigungen, wie zum Beispiel von Tensiden, wirksam gesenkt werden.

Weiters können durch die erfindungsgemäßen Maßnahmen auch staubartige Verschmutzungen aus der Prozessflüssigkeit entfernt werden. Solche Verschmutzungen können beispielsweise im Zuge der Herstellung von Behältnissen durch formgebende bzw. spanende Bearbeitungsschritte wie etwa Schneiden, Fräsen, Bohren oder dergleichen verursacht werden. Bei der Herstellung von Behältnissen kann zum Beispiel Glas- oder Metallstaub, insbesondere Aluminiumstaub anfallen, welche Stäube mit den Behältnissen in das Verfahren zur Behandlung von Lebensmitteln bzw. Behältnissen eingebracht werden können.

Schließlich kann durch die erfindungsgemäßen Maßnahmen auch eine Verschmutzung bzw. Keimbelastung der Außenseite der Behältnisse, sowie der Oberflächen der Vorrichtung zur Behandlung der Lebensmittel bzw. Behältnisse durch die Prozessflüssigkeit selbst wirksam hintangehalten werden. Dies bringt weitere Vorteile hinsichtlich allfälliger Nachbehandlungs- bzw. zusätzlicher Reinigungsmaßnahmen, deren Ausmaß zumindest verringert werden kann. Gegebenenfalls kann aufgrund der erfindungsgemäßen Maßnahmen eine Nachreinigung der Behältnisse mit Reinigungs- und Desinfektionschemikalien und/oder eine Sterilisation der Behältnisse erübrigt werden.

Die Maßnahme, die zu behandelnden Lebensmittel vor der Behandlung in Behältnisse abzufüllen, die Behältnisse verschließen, einen jeweiligen Flüssigkeitsstrom der Prozessflüssigkeit vor Zuführung in eine Behandlungszone zu temperieren, und die Behandlung der Lebensmittel in einer Behandlungszone durch Wärmeübertragung mittels einer temperierten Prozessflüssigkeit durchzuführen, indem die Prozessflüssigkeit die Außenseite der Behältnisse umströmt, stellt einerseits eine besonders effiziente Maßnahme zur Behandlung von Lebensmitteln dar, da nach der Behandlung ein bereits fertig abgefülltes Handelsprodukt bereitgestellt werden kann. Außerdem kann so ein direkter Kontakt der Lebensmittel mit der Prozessflüssigkeit vermieden werden.

Weiters kann es zweckmäßig sein, die Temperaturen der jeweiligen Flüssigkeitsströme der Prozessflüssigkeit in kontrollierter Art und Weise vor der Zuführung in eine Behandlungszone separat für jede Behandlungszone einzustellen, und die Lebensmittelprodukte in zumindest einer Behandlungszone mittels einer erhitzten Prozessflüssigkeit zu pasteurisieren. Durch die Pasteurisierung kann für die Lebensmittel eine längere Haltbarkeit erreicht werden. Die angegebenen Maßnahmen zur kontrollierten Temperierung der Prozessflüssigkeit bringen den Vorteil, dass das gesamte Verfahren zur Behandlung der Lebensmittel bzw. Behältnisse besser kontrollierbar ist. Insbesondere kann so eine ungewünschte Beschädigung der Lebensmittel und/oder der Behältnisse aufgrund zu raschen bzw. zu hohen Temperaturwechseln hintangehalten werden.

Dabei kann es besonders hinsichtlich einer Pasteurisierung der Lebensmittel sinnvoll sein, die zu behandelnden Lebensmittel, insbesondere Genussmittelprodukte, aufeinanderfolgend in zumindest einer Behandlungszone zu erwärmen, in zumindest einer Behandlungszone zu pasteurisieren, und in zumindest einer Behandlungszone abzukühlen. Einerseits kann durch eine langsame Erwärmung in zumindest einer Erwärmungszone eine schonende Erwärmung für die Lebensmittel gewährleistet werden. Durch das aktive Abkühlen in zumindest einer Abkühlzone nach dem Pasteurisieren, kann andererseits eine sogenannte "Überpasteurisierung" aufgrund hoher Temperatur der Lebensmittel für einen zu langen Zeitraum wirksam verhindert werden. Eine solche Überpasteurisierung bedingt häufig unerwünschte Veränderungen im Lebensmittel, bzw. kann den Geschmack und/oder Geruch des Lebensmittels negativ beeinflussen. Durch die angegebenen Verfahrensschritte zur sequentiellen Behandlung der Lebensmittel, insbesondere Genussmittelprodukte, ist eine gut steuerbare und für die Lebensmittel sehr schonende Verfahrensführung ermöglicht. Beispielsweise kann die Temperatur der Prozessflüssigkeit für mehrere Erwärmungszonen schrittweise erhöht werden, kann in einer oder mehreren Behandlungszonen die Prozessflüssigkeit mit einer Pasteurisierungstemperatur, zum Beispiel 80 bis 85 °C eingebracht werden, und daran anschließend kann die Prozessflüssigkeit in einer oder mehreren Kühlzonen für die Lebensmittel bzw. Behältnisse wiederum zonenweise mit einer sequentiell niedrigeren Temperatur zum Abkühlen der Lebensmittel bzw. Behältnisse, eingebracht werden.

Alternativ zu den angegebenen Temperaturbereichen, welche beispielhaft für eine Pasteurisierung von Lebensmitteln angegeben sind, können für andere Behandlungsverfahren selbstverständlich auch andere Temperaturbereiche zweckmäßig sein. Als zusätzliches Beispiel wird an dieser Stelle eine Heißdampfsterilisation angeführt, bei welcher auch Temperaturen der Prozessflüssigkeit bzw. eines Prozesswassers über 100°C angewendet werden können, sodass das Prozesswasser zumindest in Sterilisationszonen im gasförmigen Zustand auf die Behältnisse einwirkt.

Als besonders zweckmäßig hat sich erwiesen, wenn ein Flüssigkeitsstrom wenigstens einer Behandlungszone zum Erwärmen der Lebensmittel und/oder Behältnisse bei einer Temperatur zwischen 40°C und 50°C zugeführt wird. Dadurch ist eine möglichst schonende Vorerwärmung der Lebensmittel bereitgestellt, und können allzu große Temperatursprünge im Zuge einer Erwärmung der Lebensmittel verhindert werden.

In einer vorteilhaften Ausführungsform des Verfahrens kann vorgesehen sein, dass wenigstens ein mittels einer Membranfiltrationsanlage filtrierter Strom unmittelbar nachfolgend auf den Filtrationsvorgang einer UV-Bestrahlungseinrichtung zugeführt und bestrahlt wird, und ein filtrierter und bestrahlter Strom der Prozessflüssigkeit zumindest einem die Prozessflüssigkeit beinhaltenden und/oder führenden Führungselement und/oder zumindest einer Behandlungszone wieder zugeführt wird. Auf diese Weise kann gewährleistet werden, dass eine Prozessflüssigkeit mit besonders geringer Trübung mittels der UV-Bestrahlungseinrichtung bestrahlt werden kann, wodurch sich die Effizienz der UV-Bestrahlung nochmalig steigern lässt.

Zur Bereitstellung einer Prozessflüssigkeit mit geringer Trübung kann es außerdem sinnvoll sein, wenn zur Bildung wenigstens eines zu filtrierenden Stromes Prozessflüssigkeit mit einer Temperatur zwischen 40°C und 50°C herangezogen wird. Bei einer Prozessflüssigkeitstemperatur in diesem Bereich sind besonders gute Filtrationsergebnisse durch eine Membranfiltration bzw. Ultrafiltration erzielbar. Die unter anderem deshalb, da insbesondere eine Verstopfung von Filtermembranen durch Schmiermittel wie zum Beispiel Paraffine bzw. Wachse, in diesem Temperaturbereich vermieden werden kann. Solche Schmiermittel werden häufig im Zuge der Herstellung von Behältnissen benutzt, bleiben nach der Herstellung teilweise an den Behältnissen haften, und können so in die Prozessflüssigkeit eingebracht werden. Durch Membranfiltration der Prozessflüssigkeit im angegebenen Temperaturbereich kann wiederum eine Prozessflüssigkeit mit besonders geringer Trübung für die UV-Bestrahlung bereitgestellt werden.

In diesem Zusammenhang kann außerdem vorgesehen sein, dass der wenigstens eine Strom durch Entnahme der Prozessflüssigkeit aus einem temperierbaren Durchflussbehälter für die Prozessflüssigkeit gebildet wird. Dadurch kann die Effizienz der Membranfiltration bzw. die Filtrationsleistung nochmalig gesteigert werden.

Hinsichtlich der Reinigungseffizienz für die Prozessflüssigkeit im kontinuierlichen Behandlungsbetrieb ist es zweckmäßig, die im kontinuierlichen Behandlungsbetrieb aus zumindest einem die Prozessflüssigkeit beinhaltenden und/oder führenden Führungselement pro Zeiteinheit insgesamt zur Bildung wenigstens eines Stromes der Prozessflüssigkeit herangezogenen Prozessflüssigkeitsmengen so zu wählen, dass durch die Bestrahlung und/oder Filtration des Stromes bzw. der Ströme eine Entfernungsrate für Mikroorganismen erzielbar ist, welche größer ist, als die Wachstumsrate dieser Mikroorganismen in der Prozessflüssigkeit in der gleichen Zeiteinheit. Dadurch kann insbesondere erreicht werden, dass die Gesamtmenge an lebens- und vermehrungsfähigen Mikroorganismen in der Prozessflüssigkeit möglichst minimiert ist, und eine Zunahme der Gesamtmenge an Mikroorganismen in der Prozessflüssigkeit im Zuge der kontinuierlichen Behandlung der Lebensmittel und/oder Behältnisse wirksam unterbunden ist.

Überraschenderweise hat sich herausgestellt, dass pro Zeiteinheit die Bestrahlung und Membranfiltration einer verhältnismäßig kleinen Teilmenge der Prozessflüssigkeit, bezogen auf die Summe der Mengen aller pro Zeiteinheit insgesamt durch die Vorrichtung bzw. die Behandlungszonen geführten Flüssigkeitsströme der Prozessflüssigkeit, völlig genügt, um ein ausreichendes Ergebnis zu erzielen. Dadurch können die bauliche Größe und/oder die Anzahl der UV-Bestrahlungseinrichtung(en) und Membranfiltrationsanlage(n) vergleichsweise gering gehalten werden, ohne hinsichtlich Verschmutzungsgrad und Keimgehalt bzw. Keimzahl der Prozessflüssigkeit Kompromisse eingehen zu müssen. Außerdem kann so die zur Reinigung und Entkeimung der Prozessflüssigkeit aufzuwendende Energiemenge weiter reduziert werden.

Durch die Maßnahme, einen UV-bestrahlten und/oder mikro- bzw. ultrafiltrierten Strom der Prozessflüssigkeit bei Umgebungsdruck bzw. in freiem Gefälle wieder in zumindest ein die Prozessflüssigkeit beinhaltendes bzw. führendes Führungselement und/oder in eine Behandlungszone zurückzuführen, wird der Vorteil erreicht, dass ein zusätzliches Fördermittel zum Einbringen bzw. Zurückführen eines bestrahlten und/oder filtrierten Stromes in die Prozessflüssigkeit nicht benötigt wird.

Dabei kann es zweckmäßig sein, dass ein bestrahlter und/oder filtrierter Strom der Prozessflüssigkeit zumindest teilweise einem durch eine Behandlungszone bewegten Flüssigkeitsstrom der Prozessflüssigkeit nach dessen Einwirken auf die Lebensmittel bzw. die Behältnisse zugeführt wird. Diese Variante der Zuführung eines filtrierten Stromes in eine Behandlungszone ist besonders dann vorteilhaft, wenn ein Flüssigkeitsstrom der Prozessflüssigkeit unter einem gewissen Vordruck in die Behandlungszone eingebracht wird. Der Vordruck kann zum Beispiel für ein Zerstäuben der Prozessflüssigkeit notwendig sein, um die Behältnisse in der Behandlungszone möglichst gleichmäßig zu besprühen. Diese Variante des Einbringens eines filtrierten und/oder bestrahlten Stromes ist beispielsweise auch sinnvoll, um einen unerwünschten Einfluss des filtrierten und/oder bestrahlten Stromes auf die Lebensmittel bzw. Behältnisse in der Behandlungszone zu vermeiden. Dies kann zum Beispiel aufgrund eines nicht geeigneten Temperaturniveaus des filtrierten und/oder bestrahlten Stromes der Prozessflüssigkeit gegeben sein.

Es kann aber auch zweckmäßig sein, einen bestrahlten und/oder filtrierten Strom der Prozessflüssigkeit zumindest teilweise einem durch eine Behandlungszone bewegten Flüssigkeitsstrom der Prozessflüssigkeit vor dessen Einwirken auf die Lebensmittel bzw. die Behältnisse zuzuführen. Insbesondere kann so für die Behandlung der Lebensmittel bzw. Behältnisse in einer Behandlungszone eine Prozessflüssigkeit mit sehr hoher Reinheit und mit sehr geringem Keimgehalt bereitgestellt werden.

Besonders vorteilhaft kann ein zumindest teilweises Zuführen eines bestrahlten und/oder filtrierten Stromes der Prozessflüssigkeit einer im Verfahren zur Behandlung von Lebensmitteln bzw. Behältnissen zur Aufnahme der Produkte am Ende des Prozesses angeordneten Behandlungszone, zum Spülen bzw. Reinigen der Außenseite von mit dem Lebensmittelprodukt befüllten und verschlossenen Behältnissen sein. Ein solcher Verfahrensschritt wird für gewöhnlich gegen Ende eines Verfahrens zur Behandlung von Lebensmitteln bzw. Behältnissen ausgeführt, nachfolgend erfolgt allenfalls noch ein Trocknungsschritt oder sonstiger Nachbehandlungsschritt. Bei einem solchen Verfahrensschritt zum Spülen bzw. Waschen von Behältnissen ist eine Verschmutzung bzw. Verkeimung der Prozessflüssigkeit besonders kritisch, da unter Umständen Schmutz- und Keimreste, wie etwa auch Bakterien oder Bakterienreste auf der Oberfläche der Behältnisse verbleiben können. Daher ist die Zuführung eines UV-bestrahlten und/oder mittels einer Membranfiltrationsanlage filtrierten bzw. gereinigten Stromes in eine solche Behandlungszone zum Spülen von Behältnissen vorteilhaft.

Betreffend die Membranfiltration kann es es weiters zweckmäßig sein, dass ein Strom der Prozessflüssigkeit nach Filtration mittels der Membranfiltrationsanlage in einen Vorlagebehälter geführt wird, und via einem am Vorlagebehälter ausgestalteten Überlauf oder wirkungsäquivalenten Element wieder in zumindest ein die Prozessflüssigkeit beinhaltendes bzw. führendes Element und/oder in eine Behandlungszone und/oder eine UV-Bestrahlungseinrichtung zurückgeführt wird. Insbesondere wird so ein Vorrat an Prozessflüssigkeit mit hoher Reinheit gesammelt bzw. bereitgestellt, welcher für verschiedene Zwecke eingesetzt werden kann.

Beispielsweise ist eine zweckmäßige Nutzung des in einem Vorlagebehälter gesammelten Filtrats der Prozessflüssigkeit in einer Ausführungsvariante des Verfahrens dadurch erreichbar, dass in festlegbaren Zeitintervallen eine Membranfiltrationsanlage zwecks Reinigung der Filtermembranen im laufenden Betrieb vom Rest der Vorrichtung zur Behandlung von Lebensmitteln und/oder Behältnissen strömungstechnisch getrennt wird und das in dem Vorlagebehälter gesammelte Filtrat der Prozessflüssigkeit unter Umkehrung der Flussrichtung über die Filtermembranen im Vergleich zum Filtrationsbetrieb, durch eine Membranfiltrationsanlage geführt wird, die Membranfiltrationsanlage also mittels des Filtrats unter Rückspülung gereinigt wird. Im Zuge der kontinuierlichen Filtration von Teilströmen der Prozessflüssigkeit bilden sich mit der Zeit naturgemäß Rückstände an den Filtermembranen bzw. Modulen aus. Allenfalls können besonders kleine Partikel auch in eine Filtermembran bzw. einen Porenkanal einer Filtermembran eindringen und dort verbleiben. Insgesamt führen Beläge auf der Membranoberfläche und/oder in eine Membran eingedrungene und verbleibende Partikel oder Substanzen zu Verstopfungen auf und in einer Membran, und damit zu einem geringer werdenden Durchflussvermögen und zu einer Verringerung der Filtrationsleistung einer Filtermembran. Durch die angegebene, periodische Reinigung der Filtermembran-Module mittels des gesammelten Filtrats der Prozessflüssigkeit unter Umkehrung der Flussrichtung können Verstopfungen bzw. das Zuwachsen von Poren einer Membran möglichst hintangehalten werden. Dabei kann die Rückspülung durch Gaseintrag, beispielsweise durch Eintrag von Druckluft in ein Filtermembranmodul zusätzlich unterstützt werden.

Im Zuge einer Reinigung unter Umkehrung der Flussrichtung über die Filtermembranen der Membranfilteranlage fällt ein verunreinigter Flüssigkeitsabfall an. Dabei kann es sinnvoll sein, diesen Flüssigkeitsabfall unmittelbar aus der Vorrichtung zur Behandlung von Lebensmitteln bzw. Behältnissen abzuführen und durch eine entsprechende Menge an frischer Prozessflüssigkeit zu ersetzen.

Im Zusammenhang mit einer Reinigung der Filtermembranen einer Membranfiltrationsanlage durch Rückspülung, kann es außerdem zweckmäßig sein, eine UV-Bestrahlungseinrichtung zwischen einer Membranfiltrationsanlage und einem Vorlagebehälter anzuordnen. Dadurch ist eine UV-Bestrahlung der im Vorlagebehälter gesammelten Prozessflüssigkeit während eines Rückspül- bzw. Reinigungsvorgangs für eine Membranfiltrationsanlage ermöglicht, und kann zur Rückspülung der Filtermembranen eine Rückspülflüssigkeit mit besonders geringem Keimgehalt verwendet werden. Alternativ kann eine Rückspülung der Filtermembranen einer Membranfiltrationsanlage auch mittels einer extern zugeführten Spülflüssigkeit, beispielsweise Spülchemikalien-haltiges Waschwasser oder Trinkwasser durchgeführt werden.

Betreffend eine Membranfiltrationsanlage kann es zusätzlich von Vorteil sein, bei Bedarf sowohl im Behandlungsbetrieb bzw. im Filtrationsbetrieb, als auch im Reinigungsbetrieb für die Membranfiltrationsanlage, einem zu filtrierenden Strom der Prozessflüssigkeit bzw. einem filtrierten Strom oder einer in einem Vorlagebehälter gesammelten Prozessflüssigkeit mittels einer Dosiereinrichtung Chemikalien aus einer oder mehreren Chemikalienquellen beizumengen. Bei geeigneter Anordnung der Dosiereinrichtung, zum Beispiel in einem Ableitungselement für einen filtrierten Strom der Prozessflüssigkeit oder einer dem Ableitungselement zugeordneten Bypass- bzw. Rückspülleitung, kann ein Beimengen aus denselben Chemikalienquellen sowohl im Behandlungsbetrieb, als auch im Reinigungsbetrieb für die Membranfilteranlage erfolgen. Die Art und Menge einzusetzender Chemikalien richtet sich dabei nach dem jeweiligen Bedarf, und die Zuführung von Chemikalien kann von einem Bediener der Vorrichtung, bzw. einer automatischen Steuervorrichtung für die Vorrichtung, bedarfsabhängig durchgeführt werden. Dadurch, dass die Zufuhr von Chemikalien sowohl im Filtrationsbetrieb, als auch im Reinigungsbetrieb ermöglicht ist, kann ein flexibles, gezieltes Zudosieren von Chemikalien aus denselben Chemikalienquellen bedarfsabhängig durchgeführt werden. Beispiele für Chemikalien, welche sich in beiden Fällen eignen sind Tenside zu generellen Reinigung, oder Chlor zur Desinfektion der Filtermembranen oder anderen Elementen der Vorrichtung zur Behandlung von Lebensmitteln bzw. Behältnissen. Weitere, üblicherweise eingesetzte Chemikalien umfassen zum Beispiel organische Säuren zur pH-Wert-Stabilisierung, Komplexbildner und Korrosionsinhibitoren.

Zusätzlich zur Aufbereitung der Prozessflüssigkeit durch UV-Bestrahlung und Membranfiltration kann es sinnvoll sein, ein Entfernen bzw. Abscheiden gelöster bzw. suspendierter oder dispergierter Substanzen über eine Adsorptionsvorrichtung durchzuführen. Insbesondere können auf diese Weise auch unerwünschte, nicht koagulierte Bestandteile aus der Prozessflüssigkeit entfernt werden, welche durch eine Membranfiltrationsanlage nicht entfernbar sind.

Für möglichst hohe Prozesssicherheit kann es vorteilhaft sein, den Verunreinigungsgrad der Prozessflüssigkeit mittels geeigneter, in die Prozessflüssigkeit beinhaltenden und/oder führenden Führungselementen und/oder in Behandlungszonen angeordneter Sensoren kontinuierlich zu überwachen. Insbesondere können Messungen der Trübung zum Zwecke des Erfassens der Reinheit der Prozessflüssigkeit bzw. der Partikelkonzentration in der Prozessflüssigkeit dienlich sein. Die Messung der Trübung der Prozessflüssigkeit ist vor allem hinsichtlich einer Beurteilung der Effizienz der UV-Bestrahlung wichtig, da eine Trübung der Prozessflüssigkeit die Wirksamkeit der UV-Bestrahlung deutlich herabsetzen kann, wie dies obenstehend bereits erläutert wurde. Alternativ und/oder zusätzlich sind auch Messungen an Stichproben der Prozessflüssigkeit sinnvoll, besonders um Schwankungen im Gehalt an Bakterienkulturen zu erfassen bzw. Mikroorganismenwachstumsraten zu ermitteln. Durch kontinuierliche Überwachung der Trübung der Prozessflüssigkeit an verschiedenen Stellen bzw. in verschiedenen Zonen, können außerdem Verschmutzungsquellen lokalisiert werden, und gegebenenfalls gezielte Gegenmaßnahmen eingeleitet werden.

Ein weiterer Vorteil ergibt sich durch die Ausgestaltungsvariante, dass ein zu bestrahlender und/oder zu filtrierender Strom der Prozessflüssigkeit bedarfsabhängig aus unterschiedlichen, die Prozessflüssigkeit beinhaltenden bzw. führenden Führungselementen gebildet wird, mittels zumindest einer Membranfiltrationsanlage filtriert wird und ein filtrierter Strom nach dem Membranfiltrationsvorgang zumindest einem die Prozessflüssigkeit beinhaltenden bzw. führenden Führungselement und/oder zumindest einer Behandlungszone und/oder zumindest einer UV-Bestrahlungseinrichtung zugeführt wird. Insbesondere kann es vorteilhaft sein, eine Membranfiltrationsanlage mittels strömungstechnischen Schalt- und/oder Verteilerelementen derart in die Vorrichtung zur Behandlung von Produkten und/oder Behältnissen einzubinden, dass eine Membranfiltrationsanlage verschiedenen und/oder mehreren verschiedenen Führungselementen zur Beinhaltung bzw. Führung der Prozessflüssigkeit und/oder Behandlungszonen zugeordnet werden kann. Durch die Möglichkeit des eingangsseitigen Umschaltens einer Membranfiltrationsanlage auf verschiedene Quellen der Prozessflüssigkeit zur Bildung eines zu filtrierenden Stromes kann in flexibler Art und Weise auf zonenweise Schwankungen in der Qualität bzw. dem Verunreinigungsgrad der Prozessflüssigkeit, insbesondere auf Schwankungen in der Partikelkonzentration, rasch und effizient reagiert werden. Dabei besteht grundsätzlich die Möglichkeit von einer Prozessflüssigkeitsquelle auf eine andere Prozessflüssigkeitsquelle umzuschalten, also verschiedene Flüssigkeitsströme in verschiedenen Führungselementen zur Bildung des zu filtrierenden Stromes heranzuziehen. Wahlweise können aber auch mehrere Flüssigkeitsströme der Prozessflüssigkeit gleichzeitig zur Bildung eines zu filtrierenden Stromes der Prozessflüssigkeit herangezogen werden. Im diesem Fall erfolgt die Bildung des zu filtrierenden Stromes durch Mischen der herangezogenen bzw. entnommenen Teilmengen aus den verschiedenen Flüssigkeitsströmen der Prozessflüssigkeit.

Weiters kann eine Ausführungsvariante zweckmäßig sein, bei welcher Strom der Prozessflüssigkeit zur Filtration mittels einer Membranfiltrationsanlage, durch Umschalten zwischen oder Mischen von unterschiedlichen Flüssigkeitsströmen der Prozessflüssigkeit aus verschiedenen Führungselementen, in Abhängigkeit von im Zuge von Inline-Messungen und/oder Stichprobenmessungen erhaltenen Messwerten gebildet wird. Ebenso kann es von Vorteil sein, einen filtrierten Strom der Prozessflüssigkeit in Abhängigkeit von im Zuge von Inline-Messungen und/oder Stichprobenmessungen erhaltenen Messwerten, durch Einleiten oder Verteilen des filtrierten Stromes der Prozessflüssigkeit in verschiedene Flüssigkeitsströme der Prozessflüssigkeit in verschiedenen Führungselementen bzw. Behandlungszonen zurückzuführen, und/oder einer UV-Bestrahlungseinrichtung zuzuführen.

Die Aufgabe der Erfindung wird aber auch dadurch gelöst, dass eine Vorrichtung für die Behandlung von Lebensmitteln in Behältnissen zur Aufnahme von Lebensmitteln mittels einer Prozessflüssigkeit bereitgestellt wird, wie im unabhängigen Anspruch 8 definiert.

Die Vorrichtung umfasst zumindest eine Behandlungszone zum Behandeln der Lebensmittel in Behältnissen, Transportmittel zum Transportieren der Lebensmittel und/oder Behältnisse durch die Behandlungszone(n), sowie Prozessflüssigkeit beinhaltende und/oder führende Führungselemente zum Zuführen von Flüssigkeitsströmen der Prozessflüssigkeit in eine Behandlungszone und Führungselemente zum Abführen von Flüssigkeitsströmen der Prozessflüssigkeit aus einer Behandlungszone. Weiters umfasst die Vorrichtung weitere Führungselemente zur Beinhaltung und/oder Führung der Prozessflüssigkeit in der Vorrichtung und wenigstens ein Fördermittel zur Förderung von Flüssigkeitsströmen der Prozessflüssigkeit in den Führungselementen, wobei die Führungselemente derart ausgestaltet und angeordnet sind, dass die Prozessflüssigkeit zur Behandlung der Lebensmittel im Kreis wieder in die Behandlungszone oder in die Behandlungszonen geführt wird.

Insbesondere umfasst die Vorrichtung zumindest eine UV-Bestrahlungseinrichtung und zumindest eine Membranfiltrationsanlage zur Reinigung und Entkeimung der Prozessflüssigkeit durch Membranfiltration und UV-Bestrahlung. Die zumindest eine UV-Bestrahlungseinrichtung und die zumindest eine Membranfiltrationsanlage sind dabei derart mit den Führungselementen und/oder mit den Behandlungszonen strömungstechnisch leitungsverbunden, dass eine Teilmenge der pro Zeiteinheit insgesamt durch alle vorhandenen Behandlungszonen geführten Prozessflüssigkeit zur Bildung wenigstens eines Stromes der Prozessflüssigkeit herangezogen, der gebildete Strom oder die gebildeten Ströme mittels der zumindest einen Membranfiltrationsanlage filtriert und/oder mittels der zumindest einen UV-Bestrahlungseinrichtung bestrahlt, und ein filtrierter und/oder bestrahlter Strom der Prozessflüssigkeit zumindest einem Führungselement und/oder zumindest einer Behandlungszone zugeführt werden kann.

Eine derartige Vorrichtung ist in besonderem Maße für die Reinigung und Entkeimung der Prozessflüssigkeit geeignet. Damit lässt sich eine wesentliche Verbesserung gegenüber vorbekannten Vorrichtungen erreichen, welche hinsichtlich einer Entfernung von Klein- und Kleinstpartikeln sowie lebensfähiger bzw. vermehrungsfähiger Mikroorganismen bzw. Keimen aus einer zumindest teilweise im Kreis geführten Prozessflüssigkeit nur sehr eingeschränkt, oder gar nicht wirksam sind. Die Reinigung und Entkeimung mittels der oder den UV-Bestrahlungseinrichtung(en) sowie der oder den Membranfiltrationsanlage(n) kann dabei vorteilhafterweise im laufenden Behandlungsbetrieb durchgeführt werden, und ist auch vergleichsweise effizient und energiesparend.

Durch die zusätzliche Aufbereitung bzw. Reinigung der Prozessflüssigkeit mittels einer Membranfiltrationsanlage kann die Wirksamkeit bzw. Effizienz der UV-Bestrahlungseinrichtung(en) deutlich verbessert werden, da die Trübung der Prozessflüssigkeit deutlich verringert werden kann, und damit Effizienzverluste der UV-Bestrahlung durch UV-Licht-Absorption, -Streuung etc. hintangehalten werden. Des Weiteren ist eine Membranfiltrationsanlage per se wirksam in der Entfernung von Mikroorganismen, wodurch sich in synergistischer Art und Weise durch Kombination einer Membranfiltration und einer UV-Bestrahlung die Effizienz einer Verminderung vermehrungsfähiger Mikroorganismen deutlich steigern lässt. Insbesondere bei Einsatz sogenannter ,Ultrafiltration-Anlagen', also Membranfiltrationsanlagen mit Filtermembranen mit Porendurchmessern von ca. 0,2 m oder kleiner, können sowohl anorganische als auch organische Klein- und Kleinstpartikel, beispielsweise auch Bakterienkolonien, wirksam aus der Prozessflüssigkeit entfernt werden.

Sowohl eine UV-Bestrahlungseinrichtung als auch eine Membranfiltrationsanlage können eingangsseitig grundsätzlich sowohl seriell, als auch parallel mit einem die Prozessflüssigkeit beinhaltenden bzw. führenden Führungselement leitungsverbunden sein. Im Falle einer parallelen Leitungsverbindung ist es von Vorteil, eingangsseitig einer UV-Bestrahlungseinrichtung und/oder einer Membranfiltrationsanlage zumindest ein verstellbares Verteilungsmittel und/oder mehrere, zusammenwirkende Verteilungsmittel zur kontrollierten Entnahme einer festlegbaren Prozessflüssigkeitsmenge pro Zeiteinheit aus zumindest einem die Prozessflüssigkeit beinhaltenden bzw. führenden Führungselement und zur Bildung eines zu bestrahlenden und/oder filtrierenden Stromes der Prozessflüssigkeit anzuordnen. Durch dieses Ausgestaltungsmerkmal kann die pro Zeiteinheit aus einem Flüssigkeitsstrom der Prozessflüssigkeit entnommene Teilmenge sehr genau kontrolliert werden und den jeweils aktuellen Gegebenheiten angepasst werden.

Weitere sich aus den Merkmalen der Vorrichtung ergebende Vorteile wurden bereits obenstehend anhand der Beschreibung des mittels der Vorrichtung ausführbaren Verfahrens näher erläutert, weshalb an dieser Stelle eine nochmalige Erläuterung erübrigt werden kann.

Bei einer zweckmäßigen Weiterbildung der Vorrichtung umfasst diese zumindest einem Heizmittel zur Erwärmung der Prozessflüssigkeit, sowie ein Kühlmittel zur Abkühlung der Prozessflüssigkeit. Dadurch können in weiterer Folge mittels entsprechend temperierter Flüssigkeitsströme der Prozessflüssigkeit die Lebensmittel bzw. die Behältnisse gezielt erwärmt und abgekühlt werden.

Das weiterführende Merkmal, dass wenigstens eine Behandlungszone zur Applikation der Prozessflüssigkeit auf eine Außenseite von verschlossenen Behältnissen ausgestaltet ist, wobei die Prozessflüssigkeit die Außenseite eines verschlossenen Behältnisses umströmt, stellt ein besonders effizientes bauliches Merkmal für die Behandlung von Lebensmitteln dar, da nach der Behandlung ein bereits fertig abgefülltes Handelsprodukt bereitgestellt werden kann. Außerdem kann so die Gefahr einer Rekontamination der Lebensmittel in einer an die Behandlungszone(n) anschließenden Abfüllzone ausgeschlossen werden, bzw. ist eine den Behandlungszonen nachgeschaltete Abfüllzone erübrigt.

Außerdem kann es von Vorteil sein, in der Vorrichtung aufeinanderfolgend entlang einer Transportrichtung der Lebensmittel oder Behältnisse zumindest eine Behandlungszone zur Erwärmung der Lebensmittel und/oder Behältnisse, zumindest eine Behandlungszone zur Pasteurisation der Lebensmittel und zumindest eine Behandlungszone zur Abkühlung der Lebensmittel und/oder Behältnisse anzuordnen. Durch diese Ausgestaltungsform kann eine Vorrichtung zur Behandlung von Lebensmitteln in Behältnissen bereitgestellt werden, mittels welcher die Lebensmittel besonders schonend pasteurisiert werden können und eine Schädigung des pasteurisierten Lebensmittels wirksam unterbunden werden kann.

In einer vorteilhaften Ausgestaltungsvariante kann vorgesehen sein, dass wenigstens eine UV-Bestrahlungseinrichtung strömungstechnisch unmittelbar nachfolgend auf eine Membranfiltrationsanlage angeordnet ist, sodass ein filtrierter Strom der Prozessflüssigkeit unmittelbar nach erfolgter Membranfiltration mittels einer UV-Bestrahlungseinrichtung bestrahlt werden kann. Dadurch kann die Effizienz der UV-Bestrahlung nochmalig weiter gesteigert werden, da eine Prozessflüssigkeit mit besonders geringer Trübung für die UV-Bestrahlung bereitgestellt werden kann.

Des Weiteren kann vorgesehen sein, dass ein Zuleitungselement einer Membranfiltrationsanlage mit einem temperierbaren Durchflussbehälter für die Prozessflüssigkeit leitungsverbunden ist. So kann die Temperatur eines zu filtrierenden Stromes der Prozessflüssigkeit gezielt eingestellt werden, und die Filtrationseffizienz optimiert werden.

Weiterführend ist es zweckmäßig, die Vorrichtung derart auszugestalten, dass die Anzahl und Bestrahlungsleistung der UV-Bestrahlungseinrichtung(en) sowie die Anzahl und die Filtrationskapazität der Membranfiltrationsanlage(n) derart festgelegt sind, dass die im kontinuierlichen Behandlungsbetrieb aus zumindest einem die Prozessflüssigkeit beinhaltenden und/oder führenden Element pro Zeiteinheit insgesamt zur Bildung wenigstens eines Stromes der Prozessflüssigkeit herangezogene Prozessflüssigkeitsmenge so gewählt wird, dass durch die Filtration und Bestrahlung des Stromes oder der Ströme eine Entfernungsrate für Mikroorganismen erzielbar ist, welche größer ist als die Wachstumsrate der Mikroorganismen in der Prozessflüssigkeit in derselben Zeiteinheit. Durch diese Merkmale ist eine Vorrichtung geschaffen, in welcher durch die UV-Bestrahlungseinrichtung(en) und die Membranfiltrationsanlage(n) die Gesamtmenge an lebens- bzw. vermehrungsfähigen Mikroorganismen in der Prozessflüssigkeit möglichst niedrig gehalten werden kann, und eine Zunahme der Gesamtmenge an Mikroorganismen in der Prozessflüssigkeit im Zuge der kontinuierlichen Behandlung der Lebensmittel in Behältnissen wirksam unterbunden ist.

Von Vorteil kann weiters eine Ausgestaltungsform der Vorrichtung sein, bei welcher zur Rückführung eines bestrahlten und/oder filtrierten Stromes der Prozessflüssigkeit Ableitungselemente von zumindest einer UV-Bestrahlungseinrichtung und/oder von zumindest einer Membranfiltrationsanlage mit zumindest einem Führungselement und/oder zumindest einer Behandlungszone derart leitungsverbunden sind, dass ein bestrahlter und/oder filtrierter Strom der Prozessflüssigkeit dem oder den Führungselement(en) und/oder der oder den Behandlungszone(n) unter Einwirkung von Schwerkraft in freiem Gefälle zugeführt werden kann. Dadurch kann erreicht werden, dass ein zusätzliches Fördermittel zum Einbringen bzw. Zurückführen eines bestrahlten und/oder filtrierten Stromes der Prozessflüssigkeit erübrigt ist, was eine baulich einfach zu realisierende Variante und betriebs- und kosteneffiziente Variante der Vorrichtung darstellt.

Dabei kann beispielsweise zumindest eine UV-Bestrahlungseinrichtung und/oder eine Membranfiltrationsanlage ausgangseitig mit einer Öffnung in einer Behandlungszone strömungstechnisch leitungsverbunden sein, sodass ein bestrahlter und/oder filtrierter Strom der Prozessflüssigkeit in die Behandlungszone abfließen kann. Die Öffnung in einer Behandlungszone kann dabei zum Beispiel an einem oberen Ende der Behandlungszone angeordnet sein, sodass ein bestrahlter und/oder filtrierter Strom der Prozessflüssigkeit zumindest teilweise einem durch eine Behandlungszone bewegten Flüssigkeitsstrom der Prozessflüssigkeit vor dessen Einwirken auf die Lebensmittel bzw. die Behältnisse zuführbar ist. Andererseits kann es aber auch zweckmäßig sein, eine solche Öffnung an einem unteren Ende der Behandlungszone anzuordnen, um einen unerwünschten, beispielsweise thermischen Einfluss eines bestrahlten und/oder filtrierten Stromes auf die Lebensmittel bzw. Behältnisse in der Behandlungszone zu vermeiden.

Eine weitere vorteilhafte Ausgestaltungsform der Vorrichtung kann dadurch bereitgestellt werden, dass zumindest eine Behandlungszone zur Spülung der Außenseite von mit Lebensmitteln befüllten und verschlossenen Behältnissen ausgestaltet ist, welche zumindest eine Behandlungszone, bezogen auf eine Transportrichtung der Behältnisse durch die Behandlungszonen, am Ende der Behandlungszonenstrecke angeordnet ist und welche zur Spülung der Behältnisse durch Zuführung eines bestrahlten und/oder filtrierten Stromes der Prozessflüssigkeit mit zumindest einem Ableitungselement einer UV-Bestrahlungseinrichtung und/oder einem Ableitungselement einer Membranfiltrationsanlage leitungsverbunden ist. Dadurch ist eine Behandlungszone zur finalen Reinigung der Außenseite der Behältnisse mittels eines bestrahlten und/oder filtrierten Stromes der Prozessflüssigkeit bereitgestellt, in welcher die Außenseite der Behältnisse mittels einer Prozessflüssigkeit mit sehr hoher Reinheit und geringem Keimgehalt gespült werden kann.

Hinsichtlich der Membranfiltrationsanlage(n) kann auch eine Ausgestaltungsvariante von Vorteil sein, bei welcher in einem Ableitungselement einer Membranfiltrationsanlage ein Vorlagebehälter mit Überlauf ausgestaltet ist. Dadurch kann ein Vorrat an Prozessflüssigkeit gesammelt bzw. bereitgestellt werden, welcher für verschiedene Zwecke eingesetzt werden kann.

Zum Zwecke einer Reinigung einer Membranfiltrationsanlage kann es beispielsweise zweckmäßig sein, Absperrmittel in den Zuleitungselementen und den Ableitungselementen zum strömungstechnischen Abtrennen der zumindest einen Membranfiltrationsanlage vom Rest der Vorrichtung anzuordnen und in dem Vorlagebehälter und/oder einer sich zwischen dem Vorlagebehälter und dem Ableitungselement der Membranfiltrationsanlage erstreckenden Rückspülleitung zumindest ein Fördermittel anzuordnen, welches zur Förderung des in dem Vorlagebehälter gesammelten Filtrats der Prozessflüssigkeit in - verglichen mit der Flussrichtung über die Filtermembranen im Filtrationsbetrieb - gegenläufiger Richtung durch die zumindest eine Membranfiltrationsanlage ausgebildet ist. Dadurch kann im laufenden Behandlungsbetrieb der Vorrichtung zur Behandlung der Lebensmittel bzw. Behältnisse eine Membranfiltrationsanlage mittels der im Vorlagebehälter gesammelten Prozessflüssigkeit unter Rückspülung gereinigt werden. So können zum Beispiel Verstopfungen in einer Membran bzw. das Zuwachsen von Poren einer Membran möglichst hintangehalten werden, ohne dass eine Unterbrechung des Behandlungsbetriebs der Vorrichtung nötig ist.Im Zusammenhang mit einer Reinigung der Filtermembranen einer Membranfiltrationsanlage durch Rückspülung, kann es außerdem zweckmäßig sein, eine UV-Bestrahlungseinrichtung zwischen einer Membranfiltrationsanlage und einem Vorlagebehälter anzuordnen. Dadurch ist eine UV-Bestrahlung der im Vorlagebehälter gesammelten Prozessflüssigkeit während eines Rückspül- bzw. Reinigungsvorgangs für eine Membranfiltrationsanlage ermöglicht, und kann zur Rückspülung der Filtermembranen eine Rückspülflüssigkeit mit besonders geringem Keimgehalt verwendet werden.

Dabei kann es weiters zweckmäßig sein, zur Abführung des im Zuge der Reinigung unter Umkehrung der Flussrichtung über die Filtermembranen der Membranfiltrationsanlage anfallenden Flüssigkeitsabfalls, der Membranfiltrationsanlage zumindest eine absperrbare Flüssigkeitsabfallleitung zuzuordnen und in der Vorrichtung zumindest eine absperrbare Zufuhrvorrichtung zum Ersetzen des abgeführten Flüssigkeitsabfalls durch frische Prozessflüssigkeit anzuordnen. Dadurch ist es möglich, den Flüssigkeitsabfall unmittelbar aus der Vorrichtung zur Behandlung von Lebensmitteln bzw. Behältnissen abzuführen und durch eine entsprechende Menge an frischer Prozessflüssigkeit zu ersetzen.

Außerdem kann es sinnvoll sein, dass in einem Ableitungselement und/oder in der Rückspülleitung der zumindest einen Membranfiltrationsanlage eine Dosiereinrichtung angeordnet ist, mittels welcher der Prozessflüssigkeit bzw. einer in einem Vorlagebehälter gesammelten Prozessflüssigkeit sowohl im Filtrationsbetrieb, als auch im Reinigungsbetrieb für die Membranfiltrationsanlage Chemikalien aus einer oder mehreren Chemikalienquellen beigemengt werden können. Bei Anordnung einer mit der oder den Chemikalienquelle(n) leitungsverbundenen Dosiereinrichtung können so der Prozessflüssigkeit bedarfsabhängig Chemikalien, wie zum Beispiel Chlor, Tenside und andere wirksame Chemikalien sowohl im Filtrationsbetrieb, als auch im Reinigungsbetrieb einer Membranfiltrationsanlage beigemengt werden.

Zusätzlich zur Anordnung wenigstens einer UV-Bestrahlungseinrichtung und/oder einer Membranfiltrationsvorrichtung kann eine Ausgestaltungsform der Vorrichtung vorteilhaft sein, bei welcher eine Adsorptionsvorrichtung angeordnet ist. So können unerwünschte, nicht koagulierte Bestandteile ebenfalls aus der Prozessflüssigkeit entfernt werden, welche durch eine Membranfiltrationsanlage nicht entfernbar sind. Beispielsweise können Kohlenstoffverbindungen mittels Aktivkohle in einer derartigen Adsorptionsvorrichtung aus der Prozessflüssigkeit entfernt werden.

Im Hinblick auf verbesserte Prozesssicherheit kann es sinnvoll sein, dass in Führungselementen und/oder in Behandlungszonen Sensoren zur kontinuierlichen Überwachung des Verunreinigungsgrades, insbesondere durch Messen der Trübung der Prozessflüssigkeit, angeordnet sind. So kann der Verschmutzungsgrad der Prozessflüssigkeit zumindest abschnittsweise erfasst und kontinuierlich überwacht werden. Die Anordnung solcher Sensoren ist unter anderem zur Beurteilung der Effizienz der UV-Bestrahlungseinrichtung(en) hilfreich. Durch kontinuierliche Überwachung der Trübung der Prozessflüssigkeit an verschiedenen Stellen bzw. in verschiedenen Zonen der Vorrichtung zur Behandlung von Lebensmitteln und/oder Behältnissen, können außerdem Verschmutzungsquellen lokalisiert werden, und gegebenenfalls gezielte Gegenmaßnahmen eingeleitet werden.

Eine weitere, vorteilhafte Ausgestaltungsform kann dadurch gebildet sein, dass einem Zuleitungselement einer Membranfiltrationsanlage zumindest ein Umschaltmittel zugeordnet ist, welches mit wenigstens zwei verschiedenen die Prozessflüssigkeit beinhaltenden Führungselementen strömungstechnisch derart leitungsverbunden ist, dass eine Bildung des zu filtrierenden Stromes der Prozessflüssigkeit wahlweise aus einem der Flüssigkeitsströme oder mehreren Flüssigkeitsströmen der Prozessflüssigkeit in den Führungselementen oder aus mehreren Flüssigkeitsströmen der Prozessflüssigkeit erfolgen kann. Dadurch ist ein eingangsseitiges Umschalten einer Membranfiltrationsanlage auf verschiedene Quellen der Prozessflüssigkeit zur Bildung eines zu filtrierenden Stromes ermöglicht. So kann beispielsweise in flexibler Art und Weise auf zonenweise Schwankungen der Qualität bzw. des Verunreinigungsgrades der Prozessflüssigkeit, insbesondere auf Schwankungen der Partikelkonzentration, rasch und effizient reagiert werden.

Weiters kann eine Ausgestaltungsform von Vorteil sein, bei welcher einem Zuleitungselement einer Membranfiltrationsanlage zumindest ein Mischmittel zugeordnet ist, welches mit wenigstens zwei verschiedenen die Prozessflüssigkeit beinhaltenden Führungselementen strömungstechnisch derart leitungsverbunden ist, dass eine Bildung eines zu filtrierenden Stromes der Prozessflüssigkeit wahlweise aus einem der Flüssigkeitsströme oder mehreren Flüssigkeitsströmen der Prozessflüssigkeit in den Führungselementen erfolgen kann, oder die Bildung eines zu filtrierenden Stromes der Prozessflüssigkeit durch die Membranfiltrationsanlage durch Entnahme und Mischung festlegbarer Teilmengen aus mehreren Flüssigkeitsströmen der Prozessflüssigkeit erfolgen kann. Dadurch ist eine gleichzeitige Entnahme von Teilmengen an Prozessflüssigkeit aus verschiedenen Führungselementen und die Bildung eines zu filtrierenden Stromes der Prozessflüssigkeit durch Mischen der entnommenen Teilmengen an Prozessflüssigkeit ermöglicht.

Es kann aber auch zweckmäßig sein, einem Ableitungselement einer Membranfiltrationsanlage zumindest ein Umschaltelement zuzuordnen, welches mit wenigstens einem die Prozessflüssigkeit beinhaltenden Führungselement und/oder wenigstens einer Behandlungszone und/oder einer UV-Bestrahlungseinrichtung strömungstechnisch derart leitungsverbunden ist, dass die Zuführung eines filtrierten Stromes der Prozessflüssigkeit in das wenigstens eine Führungselement und/oder die wenigstens eine Behandlungszone und/oder die Bestrahlungseinrichtung kontrolliert festlegbar ist. Auf diese Weise kann ein filtrierter Strom der Prozessflüssigkeit bedarfsabhängig einem oder mehreren Führungselement(en) und/oder einer oder mehreren Behandlungszone(n) und/oder einer oder mehreren UV-Bestrahlungseinrichtungen zugeführt werden. Dies kann zum Beispiel zweckmäßig sein, um abschnittsweise gezielte Temperaturen für die Prozessflüssigkeit in der Vorrichtung zur Behandlung der Lebensmittel bzw. Behältnisse einzustellen.

Von Vorteil kann aber auch eine Ausgestaltungsvariante sein, bei welcher einem Ableitungselement einer Membranfiltrationsanlage zumindest ein Verteilerelement zugeordnet ist, welches mit wenigstens einem die Prozessflüssigkeit beinhaltenden Führungselement und/oder wenigstens einer Behandlungszone und/oder einer UV-Bestrahlungseinrichtung strömungstechnisch derart leitungsverbunden ist, dass eine Zuführung eines filtrierten Stromes der Prozessflüssigkeit in das wenigstens eine Führungselement und/oder die wenigstens eine Behandlungszone und/oder die wenigstens eine UV-Bestrahlungseinrichtung kontrolliert festlegbar ist, oder festlegbare Mengen des filtrierten Stromes der Prozessflüssigkeit in das wenigstens eine Führungselement und/oder die wenigstens eine Behandlungszone und/oder die wenigstens eine UV-Bestrahlungseinrichtung zuführbar sind.

Zum besseren Verständnis der Erfindung wird diese anhand der nachfolgenden Figuren näher erläutert. Die Erfindung ist durch die Ansprüche definiert.

Es zeigen jeweils in stark vereinfachter, schematischer Darstellung:
- Fig. 1: ein Ausführungsbeispiel einer an sich bekannten Vorrichtung zur Behandlung von Lebensmitteln und/oder Behältnissen mit Behandlungszonen, in stark vereinfachter, schematischer und unmaßstäblicher Darstellung;
- Fig. 2: ein R&I Schema einer beispielhaften Ausführungsform einer Vorrichtung zur Behandlung von Lebensmitteln und/oder Behältnissen, in stark vereinfachter Darstellung;
- Fig. 3: ausschnittsweise ein Teilschema einer Ausführungsform einer Vorrichtung mit UV-Bestrahlungseinrichtung und Membranfiltrationsanlage, in stark vereinfachter Darstellung;
- Fig. 4: ausschnittsweise ein Teilschema einer Ausführungsform einer nicht vom Schutzbereich umfassten Vorrichtung mit UV-Bestrahlungseinrichtung und Membranfiltrationsanlage, in stark vereinfachter Darstellung;
- Fig. 5: ausschnittsweise ein weiteres Teilschema einer Ausführungsform einer Vorrichtung mit einer UV-Bestrahlungseinrichtung und einer Membranfiltrationsanlage, in stark vereinfachter Darstellung;
- Fig. 6: ausschnittsweise ein weiteres Teilschema einer Ausführungsform einer Vorrichtung mit einer UV-Bestrahlungseinrichtung und einer Membranfiltrationsanlage, in stark vereinfachter Darstellung;
- Fig. 7: ausschnittsweise ein weiteres Teilschema einer Ausführungsform einer Vorrichtung mit einer UV-Bestrahlungseinrichtung und einer Membranfiltrationsanlage, in stark vereinfachter Darstellung;
- Fig. 8: ausschnittsweise ein weiteres Teilschema einer Ausführungsform einer Vorrichtung mit einer UV-Bestrahlungseinrichtung und einer Membranfiltrationsanlage, in stark vereinfachter Darstellung;
- Fig. 9: eine beispielhafte Ausgestaltungsform einer Membranfiltrationsanlage mit nachgeschalteter UV-Bestrahlungseinrichtung, schematisch und in stark vereinfachter Darstellung;
- Fig. 10: ausschnittsweise ein weiteres Teilschema einer Ausführungsform einer Vorrichtung mit einer UV-Bestrahlungseinrichtungen und einer Membranfiltrationsanlage, in stark vereinfachter Darstellung;
- Fig. 11: ausschnittsweise ein weiteres Teilschema einer Ausführungsform einer Vorrichtung mit einer UV-Bestrahlungseinrichtung und einer Membranfiltrationsanlage, in stark vereinfachter Darstellung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

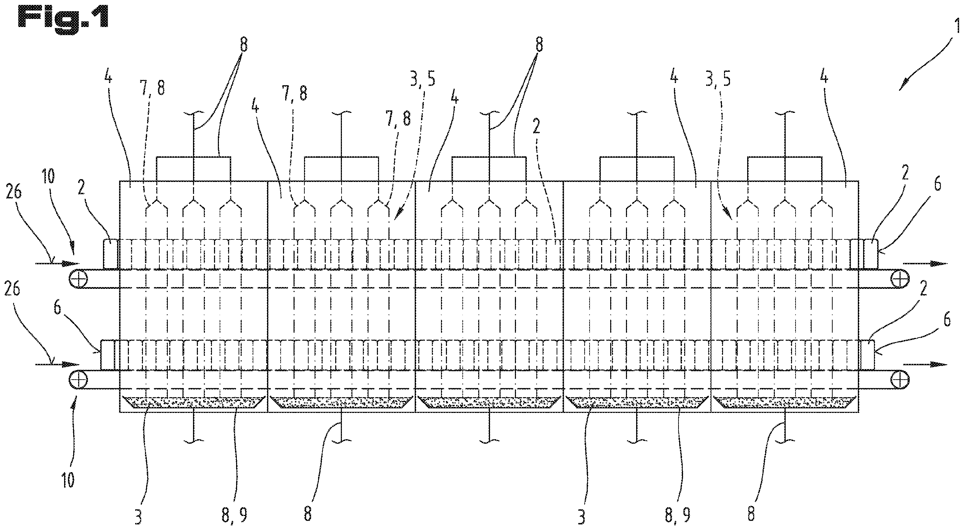

In der Fig. 1 ist ein Beispiel für eine Anordnung von Behandlungszonen einer Vorrichtung 1 zur Behandlung von Lebensmitteln und/oder für die Behandlung von Behältnissen 2 zur Aufnahme von Lebensmitteln, in schematischer und stark vereinfachter Darstellungsweise, gezeigt. Dabei werden die Lebensmittel bzw. die Behältnisse 2 mittels einer Prozessflüssigkeit 3 in zumindest einer Behandlungszone 4 behandelt. Im in Fig. 1 gezeigten Ausführungsbeispiel sind die zu behandelnden Lebensmittel in verschlossenen Behältnissen 2 befindlich, und werden mittels der Prozessflüssigkeit 3 dadurch behandelt, dass die Außenseite 6 der Behältnisse 2 durch einen Flüssigkeitsstrom 5 der Prozessflüssigkeit 3 umströmt wird. Der Flüssigkeitsstrom 5 der Prozessflüssigkeit 3 durch eine Behandlungszone 4 wird im in Fig. 1 dargestellten Ausführungsbeispiel dadurch generiert, dass die Prozessflüssigkeit 3 mittels Verteilungsvorrichtungen, wie zum Beispiel Sprühdüsen 7, an der Oberseite einer Behandlungszone 4 verteilt wird, und der Flüssigkeitsstrom 5 der Prozessflüssigkeit 3 die Behandlungszonen 4 von oben nach unten durchquert. Einer Behandlungszone 4 wird ein Flüssigkeitsstrom 5 der Prozessflüssigkeit 3 mittels baulich geeigneten Führungselementen 8, wie etwa mit den Sprühdüsen 7 leitungsverbundenen Rohrleitungen, zugeführt. In analoger Art und Weise wird ein Flüssigkeitsstrom 5 der Prozessflüssigkeit 3 nach Durchtritt durch eine Behandlungszone 4 mittels weiterer, einem unteren Bereich einer Behandlungszone 4 zugeordneter Führungselemente 8, wieder aus einer Behandlungszone 4 entfernt. Die zur Ableitung eines Flüssigkeitsstromes 5 der Prozessflüssigkeit 3 aus einer Behandlungszone 4 vorgesehenen Führungselemente 8 können beispielsweise durch Sammelwannen 9 gebildet sein, welche den durch eine Behandlungszone 4 rieselenden Flüssigkeitsstrom 5 der Prozessflüssigkeit 3 am unteren Ende der Behandlungszone 4 sammelt. Die mittels der Sammelwannen 8, 9 aufgefangene Prozessflüssigkeit 3 kann dann mittels mit den Sammelwannen 8, 9 leitungsverbundenen Führungselementen 8, bzw. Rohrleitungen 8 aus einer Behandlungszone 4 abgeführt werden, wie dies schematisch in Fig. 1 angedeutet ist.

Die Behältnisse 2 können mittels geeigneter Transportmittel 10, wie zum Beispiel Förderbändern oder dergleichen, durch die Behandlungszonen 4 hindurchtransportiert werden, beispielsweise in zwei Ebenen von links nach rechts, wie in Fig. 1 durch die Pfeile 26, welche eine Transportrichtung 26 für die Behältnisse 2 veranschaulichen, angedeutet ist.

Im in Fig. 1 gezeigten Ausführungsbeispiel können die beiden auf der in Fig. 1 linken Seite dargestellten Behandlungszonen 4, beispielsweise zur sukzessiven Erwärmung der Behältnisse 2 bzw. in den Behältnissen befindlichen Lebensmittel herangezogen werden. In weiterer Folge kann die in Fig. 1 mittig dargestellte Behandlungszone 4 beispielsweise zur Pasteurisierung der Lebensmittel benutzt werden und die beiden in Fig. 1 auf der rechten Seite dargestellten Behandlungszonen 4 zur sequentiellen Abkühlung der Lebensmittel bzw. Behältnisse benutzt werden. Die entsprechenden Behandlungsschritte zur Erwärmung, Pasteurisierung und Abkühlung können dabei durch die Zuführung eines jeweils geeignet temperierten Flüssigkeitsstromes 5 der Prozessflüssigkeit 3 in die jeweilige Behandlungszone 4 erfolgen. Dabei kann es zweckmäßig sein, dass ein Flüssigkeitsstrom 5 wenigstens einer Behandlungszone 4 zum Erwärmen der Lebensmittel und/oder Behältnisse bei einer Temperatur zwischen 40°C und 50°C zugeführt wird.

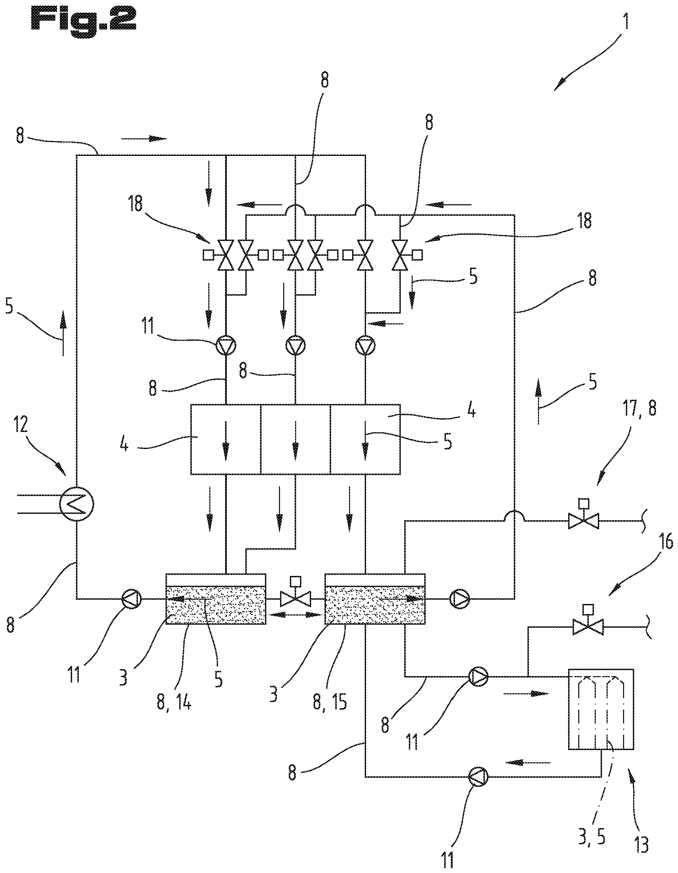

Zum Zweck der Zuführung eines Flüssigkeitsstromes 5 der Prozessflüssigkeit 3 in die jeweilige Behandlungszone 4, können den Behandlungszonen 4 jeweils Fördermittel 11 zugeordnet sein, wie dies aus dem in Fig.2 dargestellten Fließschema ersichtlich ist. Um unnötige Wiederholungen zu vermeiden, werden in der Fig. 2 für gleiche Teile gleiche Bezugszeichen bzw. Bauteilbezeichnungen wie in den vorangegangenen Fig. 1 verwendet, wobei in der Fig. 2 zur besseren Übersichtlichkeit nur drei Behandlungszonen 4 dargestellt sind. Die in Fig. 2 links dargestellte Behandlungszone 4 kann dabei beispielsweise wiederum zur Erwärmung der Behältnisse bzw. Lebensmittel benutzt werden, während die in der Fig. 2 mittig gezeichnete Behandlungszone 4 zur Pasteurisierung und die in der Fig. 2 rechts gezeichnete Behandlungszone 4 zur Abkühlung der Behältnisse bzw. Lebensmittel vorgesehen sein kann.

Das in Fig. 2 dargestellte Ausführungsbeispiel eines Fließschemas einer Vorrichtung 1 umfasst ein Heizmittel 12 zur Erwärmung die Prozessflüssigkeit 3, sowie ein Kühlmittel 13 zur Abkühlung der Prozessflüssigkeit 3. Im Falle des in Fig. 2 gezeigten Ausführungsbeispiels wird die Prozessflüssigkeit 3 dem Heizmittel 12 mittels eines weiteren Fördermittels 11 aus einem als Flüssigkeitstank 14 ausgestalteten Führungselement 8 via als Rohrleitungen oder dergleichen ausgestalteten Führungselementen 8 zugeführt, bzw. durch das Heizmittel 12 hindurchgeführt. Die Erwärmung der Prozessflüssigkeit 3 im Heizmittel 12 kann auf verschiedenste Art und Weise erfolgen, beispielsweise durch Wärmeübertragung auf die Prozessflüssigkeit durch ein Heizmedium, zum Beispiel Sattdampf. Im Prinzip kann jegliche Wärmequelle zur Erhitzung der Prozessflüssigkeit 3 genutzt werden, wobei es zum Zweck einer Pasteurisierung von Lebensmitteln zweckmäßig sein kann, dass das Heizmittel 12 zur Erwärmung der Prozessflüssigkeit 3 auf eine Temperatur von zumindest 80 °C ausgebildet ist. Der so erwärmte Flüssigkeitsstrom 5 der Prozessflüssigkeit 3 kann nach Durchleiten durch das Heizmittel 12 den Behandlungszonen 4 über Führungselemente 8, zum Beispiel Rohrleitungen, zugeführt werden.

Alternativ oder zusätzlich zu dem in Fig. 1 und Fig. 2 dargestellten Ausführungsbeispielen sind auch andere Methoden zur Behandlung der Lebensmittel bzw. Behältnisse denkbar. Beispielsweise kann eine Prozessflüssigkeit, insbesondere Prozesswasser zur Behandlung von Lebensmitteln und/oder Behältnissen auch über den Siedepunkt des Prozesswassers, also auf eine Temperatur über 100 °C erhitzt werden, und einer Behandlungszone als Heißdampf zugeführt werden. Dies ist zum Beispiel zum Zwecke einer Sterilisation zweckmäßig.

Zur Abkühlung der Prozessflüssigkeit 3 kann die Prozessflüssigkeit 3 dem Kühlmittel 13 wie in Fig. 2 dargestellt, zum Beispiel aus einem Flüssigkeitstank 15 zugeführt werden. Im in Fig. 2 gezeigten Ausführungsbeispiel ist das Kühlmittel 13 mit dem Flüssigkeitstank, zum Beispiel einem Kaltwassertank 15 über Führungselemente 8 derart leitungsverbunden, dass ein Flüssigkeitsstrom 5 der Prozessflüssigkeit 3 mittels eines Fördermittels 11 aus dem Flüssigkeitstank 15 entnehmbar ist und nach erfolgter Kühlung der Flüssigkeitsstrom 5 der Prozessflüssigkeit 3 wieder in den Flüssigkeitstank 15 rückführbar ist. Das Kühlmittel 13 kann dabei beispielsweise als Kühlturm oder Wärmetauscher ausgeführt sein, in welchem die Prozessflüssigkeit 3 durch in Gegenrichtung strömende Luft oder ein anderes Kühlmedium gekühlt wird.

Wie weiters aus Fig. 2 ersichtlich ist, sind die Führungselemente 8 zur Beinhaltung bzw. Führung der Prozessflüssigkeit 3 bzw. Flüssigkeitsströmen 5 der Prozessflüssigkeit 3 in der Vorrichtung 1 derart ausgestaltet bzw. angeordnet, dass die Prozessflüssigkeit 3 zumindest teilweise im Kreis wieder in die Behandlungszonen 4 zurückgeführt werden kann. Zur besseren Veranschaulichung sind in Fig. 2 die Strömungsrichtungen für die Flüssigkeitsströme 5 der Prozessflüssigkeit 3 für den Behandlungsbetrieb der Vorrichtung 1 mittels der Pfeile angedeutet. Zur Abführung einer Teilmenge der Prozessflüssigkeit 3 sind absperrbare Entleerungsvorrichtungen 16 vorgesehen und zur Zuführung frischer Prozessflüssigkeit 3 ist zumindest ein absperrbares, als Zufuhrvorrichtung 17 ausgestaltetes, Führungselement 8 angeordnet. Im in Fig. 2 gezeigten Ausführungsbeispiel sind in den jeweils eingangsseitig an den Behandlungszonen 4 angeordneten Führungselementen 8 Durchflussregeleinrichtungen 18 vorgesehen, mittels welcher Durchflussregeleinrichtungen 18 die Flüssigkeitsströme 5 der Prozessflüssigkeit 3 in unterschiedliche Temperaturniveaus kontrolliert abgemischt werden können.

Dadurch ist eine gezielte Einstellung der Temperatur der Flüssigkeitsströme 5 der Prozessflüssigkeit 3 für jede Behandlungszone 4 separat ausführbar. Anstelle der dargestellten Durchflussregeleinrichtungen 18 können auch Drei-Weg-Mischventile oder andere geeignete Mittel zur kontrollierten Abmischung bzw. Einstellung der Temperatur eines Flüssigkeitsstromes 5 der Prozessflüssigkeit 3 angeordnet sein.

Selbstverständlich stellt das in Fig. 2 gezeigte Ausführungsbeispiel lediglich eine Ausgestaltungsform einer Vorrichtung 1 zur Behandlung von Produkten bzw. Behältnissen dar. Beispielsweise ist es bei einigen Ausführungsformen für Vorrichtungen zur Behandlung von Lebensmitteln und/oder Behältnissen üblich, einen Flüssigkeitsstrom nach Ableitung aus einer Behandlungszone direkt einer anderen Behandlungszone zuzuführen. Dies ist zum Beispiel dann zweckmäßig, wenn ein aus einer Behandlungszone abgeleiteter Flüssigkeitsstrom der Prozessflüssigkeit ein zum Behandeln der Lebensmittel und/oder Behältnisse in einer anderen Behandlungszone geeignetes Temperaturniveau aufweist. Solche alternativen oder ergänzenden Ausgestaltungsformen zu dem in Fig. 2 gezeigten Ausgestaltungsbeispiel können bedarfsabhängig von einem auf dem jeweiligen Gebiet tätigem Fachmann vorgenommen werden, bzw. sind aus dem Stand der Technik hinlänglich bekannt, sodass auf Darlegung weiterer Ausführungsbeispiele an dieser Stelle verzichtet werden kann.

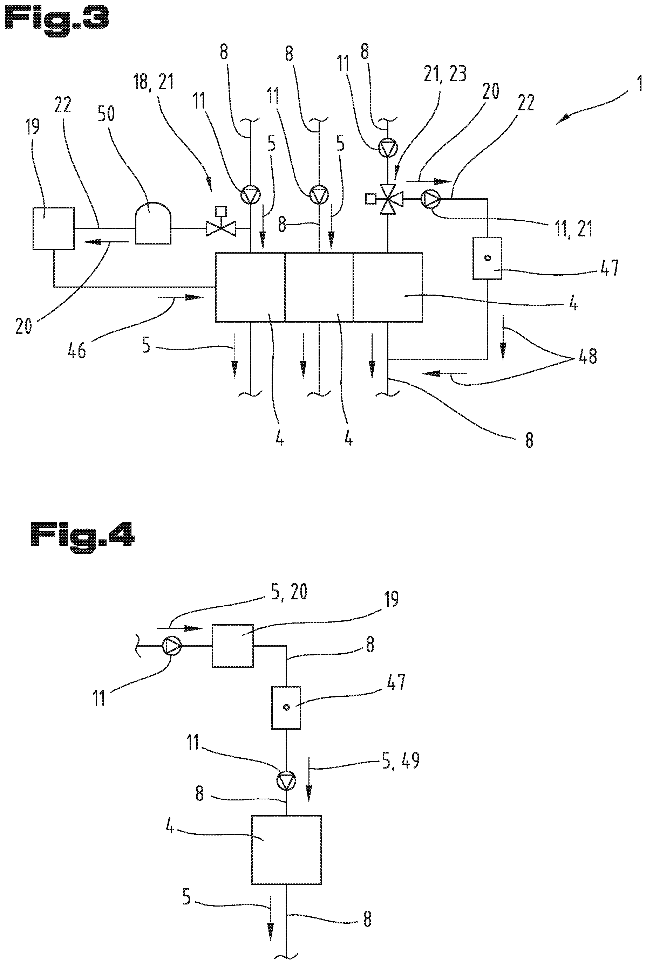

In der Fig. 3 ist ein Schema einer Vorrichtung 1 zur Behandlung von Lebensmitteln und/oder Behältnissen ausschnittsweise dargestellt, wobei in der Vorrichtung 1 wenigstens eine Membranfiltrationsanlage 19 und wenigstens eine UV-Bestrahlungseinrichtung 47 zur Reinigung und Entkeimung der Prozessflüssigkeit vorgesehen ist. In Fig. 3 werden für gleiche Teile gleiche Bezugszeichen bzw. Bauteilbezeichnungen, wie in den vorangegangenen Fig. 1 und 2 verwendet. Um unnötige Wiederholungen zu vermeiden, wird auf die detaillierte Beschreibung in den bzw. zu den vorangegangenen Fig. 1, 2 hingewiesen bzw. Bezug genommen.

Die in der Fig. 3 beispielhaft dargestellten UV-Bestrahlungseinrichtung 47 und Membranfiltrationsanlage 19 sind derart mit den Führungselementen 8 und/oder mit den Behandlungszonen 4 strömungstechnisch leitungsverbunden, dass zumindest eine Teilmenge oder die gesamte Menge der pro Zeiteinheit insgesamt durch alle vorhandenen Behandlungszonen 4 geführten Prozessflüssigkeit zur Bildung wenigstens eines zu filtrierenden und/oder zu bestrahlenden Stromes 20, 20 der Prozessflüssigkeit herangezogen werden kann, der gebildete Strom 20 oder die gebildeten Ströme 20, 20 mittels der zumindest einen Membranfiltrationsanlage 19 filtriert und/oder mittels der zumindest einen UV-Bestrahlungseinrichtung 47 bestrahlt, und ein filtrierter und/oder bestrahlter Strom 46, 48 der Prozessflüssigkeit zumindest teilweise einem Führungselement 8 und/oder einer Behandlungszone 4 wieder zugeführt werden kann.

Im Prinzip kann jeder beliebige Flüssigkeitsstrom 5 der Prozessflüssigkeit zur Bildung eines zu filtrierenden und/oder zu bestrahlenden Stromes 20 der Prozessflüssigkeit herangezogen werden, bzw. können Teilmengen der Prozessflüssigkeit aus jedem beliebigen Flüssigkeitsstrom 5 zur Bildung eines Stromes 20 entnommen werden. Ebenso kann die Rückführung eines filtrierten und/oder bestrahlten Stromes 46, 48 der Prozessflüssigkeit im Prinzip in jedes beliebige Führungselement 8 für die Prozessflüssigkeit und/oder in jede beliebige Behandlungszone 4 erfolgen. Allerdings ergeben sich bei bestimmten Anordnungsvarianten der Einbindung einer oder mehrerer Membranfiltrationsanlage(n) 19 bzw. UV-Bestrahlungseinrichtungen(en) 47 Vorteile, welche nachstehend anhand weiterer, mittels Figuren dargestellter Ausführungsbeispiele noch näher erläutert werden.

Im ausschnittsweisen dargestellten Ausführungsbeispiel gemäß Fig. 3 wird ein gebildeter Strom 20 einer Membranfiltrationsanlage 19 zugeführt (links in Fig. 3), und ein gebildeter Strom 20 einer UV-Bestrahlungseinrichtung 47 zugeführt (rechts in Fig. 3).

Die in der Fig. 3 beispielhaft dargestellte Membranfiltrationsanlage 19 ist dabei Bypass-artig zwischen einem einen Flüssigkeitsstrom 5 führenden Führungselement 8 und einer Behandlungszone 4 angeordnet. Die beispielhaft dargestellte UV-Bestrahlungseinrichtung 47 ist Bypass-artig zwischen zwei Führungselementen 8 angeordnet. Im in der Fig. 3 dargestellten Ausführungsbeispiel wird ein filtrierter Strom 46 einer Behandlungszone 4 zugeführt, und ein bestrahlter Strom 48 einem Führungselement 8 zugeführt. Selbstverständlich wäre es auch möglich einen filtrierter Strom einem Führungselement 8 und einen bestrahlten Strom 48 einer Behandlungszone 4 zuzuführen.

Im dem in Fig. 3 gezeigten Ausführungsbeispiel wird mittels geeigneter Verteilungsmittel 21 pro Zeiteinheit jeweils eine Teilmenge aus einem Flüssigkeitsstrom 5 der Prozessflüssigkeit zur Bildung eines zu filtrierenden bzw. zu bestrahlenden Stromes 20 entnommen. Zur kontrollierten bzw. steuerbaren Entnahme einer Teilmenge aus dem Flüssigkeitsstrom 5 zur Bildung eines Stromes 20 kann in einem Zuleitungselement 22 zu einer Membranfiltrationsanlage 19 bzw. zu einer UV-Bestrahlungseinrichtung 47 beispielsweise ein als Durchflussregeleinrichtung 18 ausgestaltetes Verteilungsmittel 21 angeordnet sein, wie dies auf der linken Seite in Fig. 3 gezeigt ist. Alternativ kann zum Beispiel, wie auf der rechten Seite der Fig. 3 dargestellt, auch ein Drei-Wege-Verteilerventil 23 als Verteilungsmittel 21 benutzt werden. Dabei kann auch zusätzlich ein mit einem Ventil 18, 23 zusammenwirkendes, weiteres Verteilungsmittel 21 in Form eines Fördermittels 11 bzw. einer Pumpe ausgestaltet sein, um eine kontrollierte Entnahme einer Teilmenge der Prozessflüssigkeit aus dem Führungselement 8 zu ermöglichen. Bevorzugt wird allerdings auf das Anordnen eines zusätzlichen Fördermittels 11 in einem Zuleitungselement 22 einer UV-Bestrahlungseinrichtung 47 bzw. einer Membranfiltrationsanlage 19 verzichtet und, wie auf der linken Seite in Fig. 3 dargestellt, die Entnahme einer Teilmenge der Prozessflüssigkeit mittels lediglich einem in einem Führungselement 8 der Vorrichtung 1 angeordnetem Fördermittels 11 bewerkstelligt.

Die in Fig. 3 auf der linken Seite dargestellte Behandlungszone 4 kann beispielsweise wiederum als Aufwärmzone für die Lebensmittel bzw. Behältnisse ausgebildet sein, die in Fig. 3 mittig dargestellte Behandlungszone 4 zur Pasteurisierung der Lebensmittel und die in Fig. 3 rechts gezeichnete Behandlungszone 4 zur Abkühlung der Lebensmittel bzw. Behältnisse dienen. Dementsprechend würde im laufenden Behandlungsbetrieb der Vorrichtung 1 der mittig angeordneten Pasteurisierungszone 4 ein Flüssigkeitsstrom 5 mit hoher Temperatur der Prozessflüssigkeit 3 zugeleitet, wohingegen den Behandlungszonen 4, zur Erwärmung bzw. Abkühlung der Lebensmittel bzw. Behältnisse, Flüssigkeitsströme 5 mit vergleichsweise niedriger Temperatur zugeführt werden.

Wie in Fig. 3 angedeutet ist, kann zur Schonung einer Membranfiltrationsanlage 19 ein Flüssigkeitsstrom 5 mit verhältnismäßig geringer Temperatur zur Bildung eines zu filtrierenden Stromes 20 der Prozessflüssigkeit herangezogen werden. Im in Fig. 3 dargestellten Ausführungsbeispiel sind daher Zuleitungselemente 22 der dargestellten Membranfiltrationsanlage 19 mit den zu der Aufwärmungs-Behandlungszone, also der links in Fig. 3 dargestellten Behandlungszone 4, führenden Führungselementen 8 strömungstechnisch leitungsverbunden. Diese Führungselemente 8 beinhalten einen Flüssigkeitsstrom 5 mit verhältnismäßig geringer Temperatur der Prozessflüssigkeit. Bevorzugt ist die zumindest eine Membranfiltrationsanlage 19 zur Bildung eines zu filtrierenden Stromes 20 der Prozessflüssigkeit an solchen Stellen mit Führungselementen 8 der Vorrichtung 1 strömungstechnisch leitungsverbunden ist, dass zur Bildung wenigstens eines zu filtrierenden Stromes 20 Prozessflüssigkeit mit einer Temperatur zwischen 40°C und 50°C herangezogen wird. Wie sich gezeigt hat, ist die Membranfiltration bzw. die Filtrationsleistung eines Stromes 20 einer Prozessflüssigkeit in diesem Temperaturbereich besonders effizient.

Wie weiters in der Fig. 3 dargestellt ist, kann hierbei zusätzlich vorgesehen sein, dass ein Zuleitungselement 22 einer Membranfiltrationsanlage 19 mit einem temperierbaren Durchflussbehälter 50 für die Prozessflüssigkeit leitungsverbunden ist. Ein derartiger Durchflussbehälter 50 kann beispielsweise als Pufferspeicher mit integriertem Wärmetauscher oder als Pufferspeicher mit Elektroheizung usw. ausgebildet sein. Auf diese Weise kann ein Strom 20 durch Entnahme der Prozessflüssigkeit aus dem temperierbaren Durchflussbehälter 50 für die Prozessflüssigkeit gebildet werden.

Alternativ zum in Fig. 3 gezeigten Ausführungsbeispiel kann zur Bildung eines Stromes 20 der Prozessflüssigkeit, ein gesamter Flüssigkeitsstrom 5 der Prozessflüssigkeit herangezogen werden, wie dies in Fig. 4 schematisch dargestellt ist. Im in Fig. 4 dargestellten Beispiel sind eine Membranfiltrationsanlage 19 und eine UV-Bestrahlungseinrichtung 47 strömungstechnisch seriell in einem zu einer Behandlungszone 4 führenden Führungselement 8 angeordnet. Daher wird der gesamte durch das Führungselement 8 geführte Flüssigkeitsstrom 5 der Prozessflüssigkeit 3 durch die dargestellte Membranfiltrationsanlage 19 und die dargestellte UV-Bestrahlungseinrichtung 47 geleitet und im in Fig. 4 dargestellten Beispiel nach erfolgter Membranfiltration und UV-Bestrahlung einer Behandlungszone 4 zugeführt. Wie in Fig. 4 dargestellt, kann bei einer derartigen Anordnung aufgrund des Druckverlustes über die Membranfiltrationsanlage 19 und die UV-Bestrahlungseinrichtung 47 ein Anordnen eines zusätzlichen Fördermittels 11 zur Einbringung der Prozessflüssigkeit 3 in eine Behandlungszone 4 nach erfolgter Membranfiltration und UV-Bestrahlung erforderlich sein.

Eine zur Durchführung des Verfahrens bzw. zur Verwendung in der Vorrichtung geeignete UV-Bestrahlungseinrichtung kann grundsätzlich auf verschiedene Arten ausgestaltet sein. Als allgemein bekannt wird hierbei vorausgesetzt, dass UV-Bestrahlungseinrichtungen mit Strahlungsquellen, welche UV-Licht mit einer Wellenlänge von bzw. kleiner als 254 nm abstrahlen bzw. umfassen, besonders wirksam sind. Insbesondere werden durch dieses sogenannte UVC-Licht molekulare Bindungen in der DNA von Mikroorgansimen aufgebrochen, wodurch die Mikroorganismen abgetötet oder zumindest in einen harmlosen, nicht vermehrungsfähigen Zustand übergeführt werden können. Als UVC-Strahlungsquelle(n) werden häufig Quecksilberdampflampen oder Amalgam-Lampen verwendet.

Bevorzugt werden UV-Bestrahlungseinrichtungen in der Vorrichtung angeordnet, welche zum Durchströmen der zu bestrahlenden bzw. zu entkeimenden Flüssigkeit vorgesehen sind, also als Durchflusseinrichtungen ausgestaltet sind. Derartige UV-Bestrahlungseinrichtungen können beispielsweise eine chemikalienresistente Ummantelung, beispielsweise aus rostfreien Edelstahl umfassen, innerhalb welcher die UVC-Strahlungsquelle(n) angeordnet sind. Die Ummantelung kann dabei wenigstens ein Zuleitungselement und wenigstens ein Ableitungselement umfassen, sodass die zu entkeimende Flüssigkeit durch das Innere der UV-Bestrahlungseinrichtung bzw. den durch die Ummantelung definierten Innenraum geleitet und bestrahlt werden kann. Die Strahlungsquelle(n), beispielsweise Mitteldruck-Quecksilberdampflampe(n) kann bzw. können zum Beispiel in einer Quarzglashülse im durch die Ummantelung definierten Innenraum der UV-Bestrahlungseinrichtung angeordnet sein, sodass die zu bestrahlende Flüssigkeit die Strahlungsquelle(n) umströmt. Alternativ kann auch vorgesehen sein, dass die zu bestrahlende Flüssigkeit im Innenraum einer UV-Bestrahlungseinrichtung in einem oder mehreren UV-transparenten Führung(en) geführt werden, und von außen durch die Strahlungsquelle(n) bestrahlt wird. Derartige UV-Bestrahlungseinrichtungen sind im Prinzip aus dem Stand der Technik bekannt.

Wichtig ist hierbei, dass die Bestrahlungsleistung einer UV-Bestrahlungseinrichtung derart ausgewählt wird, dass eine wirksame, keimreduzierende Dosis der UVC-Strahlung in die zu bestrahlende Flüssigkeit eingebracht werden kann. Die Wirksamkeit einer UV-Bestrahlungseinrichtung hinsichtlich keimreduzierender Wirkung ist direkt von der eingebrachten UV-Dosis abhängig. Die UV-Licht-Dosis ist das Produkt aus UV-Licht-Intensität und Bestrahlungszeit. Damit ist die UV-Dosis einer UV-Bestrahlungseinrichtung unter anderem abhängig von Faktoren wie Durchflussrate bzw. -geschwindigkeit der Flüssigkeit durch die UV-Bestrahlungseinrichtung und UV-Lichtdurchlässigkeit, sowie Trübung der Flüssigkeit. Hinsichtlich Langzeitwirkung sind aber auch die Bildung von Ablagerungen an der Strahlungsquelle, sowie auch die abnehmende Strahlungsintensität mit steigendem Lampenalter zu berücksichtigen. Daher kann es zweckmäßig sein, wenn die UV-Bestrahlungseinrichtung Überwachungsvorrichtungen umfasst, welche die Strahlungsleistung bzw. -intensität der Strahlungsquelle(n) überwachen, sodass eine Strahlungsquelle bei nicht mehr ausreichender Strahlungsintensität ausgewechselt werden kann.

Hinsichtlich der Eindringtiefe der UVC-Strahlung in die zu bestrahlende Flüssigkeit, können im Innenraum der Ummantelung einer UV-Bestrahlungseinrichtung beispielsweise Elemente vorgesehen sein, durch welch ein durch die UV-Bestrahlungseinrichtung geführter Strom manipuliert werden kann. Zum Beispiel kann eine Aufteilung eines durch eine UV-Bestrahlungseinrichtung geführten Stromes zweckmäßig sein, oder auch eine spezielle Führung durch Umlenkungselemente im Innenraum der Ummantelung der UV-Bestrahlungseinrichtung. Weiters können Reflexionselemente zur besseren Verteilung der UV-Strahlung in der durchströmenden Flüssigkeit zweckmäßig sein.

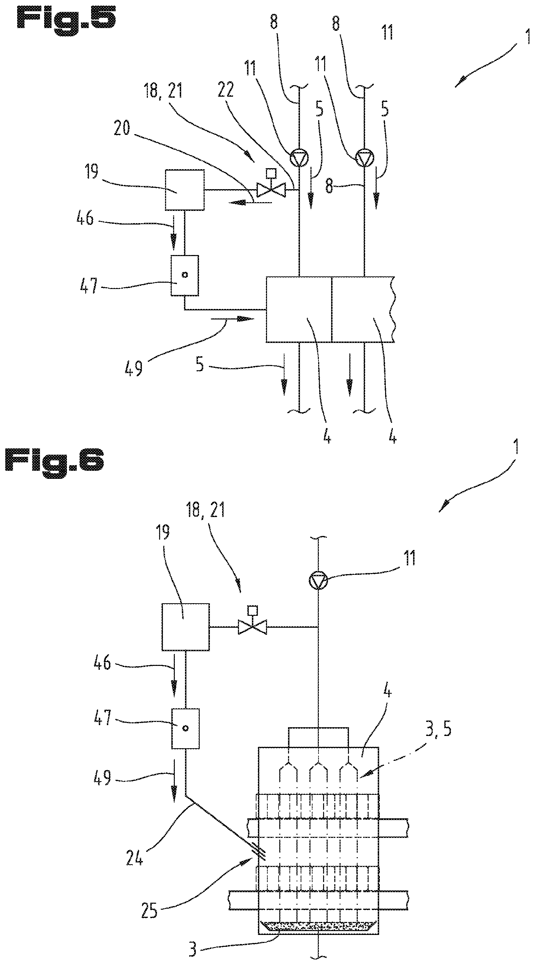

In der Fig. 5 ist ein weiteres Ausführungsbeispiel für eine Vorrichtung 1 zur Behandlung von Lebensmitteln und/oder Behältnissen dargestellt, wobei wiederum für gleiche Teile gleiche Bezugszeichen bzw. Bauteilbezeichnungen, wie in den vorangegangenen Fig. 1 bis 4 verwendet werden. Um unnötige Wiederholungen zu vermeiden, wird auf die detaillierte Beschreibung in den vorangegangenen Fig. 1 bis 4 hingewiesen bzw. Bezug genommen. In der Fig. 5 ist eine UV-Bestrahlungseinrichtung 47 strömungstechnisch unmittelbar nachfolgend auf eine Membranfiltrationsanlage 19 angeordnet. Auf diese Weise kann ein filtrierter Strom 46 unmittelbar nachfolgend auf den Filtrationsvorgang einer UV-Bestrahlungseinrichtung 47 zugeführt und bestrahlt werden. Ein filtrierter und bestrahlter Strom 49 der Prozessflüssigkeit kann anschließend wiederum zumindest einem die Prozessflüssigkeit beinhaltenden und/oder führenden Führungselement 8 und/oder zumindest einer Behandlungszone 4 wieder zugeführt werden. Im Ausführungsbeispiel gemäß der Fig. 5 wird ein filtrierter und bestrahlter Strom 49 einer Behandlungszone 4 zugeführt.

Bevorzugt werden die Anzahl und Bestrahlungsleistung der UV-Bestrahlungseinrichtung(en) 47sowie die Anzahl und die Filtrationskapazität der Membranfiltrationsanlage(n) 19 in der Vorrichtung 1 derart festgelegt bzw. ausgestaltet, dass die im kontinuierlichen Behandlungsbetrieb aus zumindest einem die Prozessflüssigkeit beinhaltenden und/oder führenden Führungselement 8 pro Zeiteinheit insgesamt zur Bildung wenigstens eines Stromes 20 der Prozessflüssigkeit 3 herangezogene Prozessflüssigkeitsmenge so gewählt werden kann, dass durch die Filtration und UV-Bestrahlung des Stromes 20 oder der Ströme 20 eine Entfernungsrate für Mikroorganismen erzielbar ist, welche größer ist, als die Wachstumsrate dieser Mikroorganismen in der Prozessflüssigkeit 3 im gleichen Zeitintervall bzw. derselben Zeiteinheit.

Bevorzugt wird eine Zuführung eines bestrahlten und/oder filtrierten Stromes 46, 48, 49 der Prozessflüssigkeit in eine Behandlungszone 4 und/oder ein Führungselement 8 ohne Fördermittel 11 bewerkstelligt. Dazu kann es zweckmäßig sein, dass Ableitungselemente 24 einer UV-Bestrahlungseinrichtung 47 und/oder einer Membranfiltrationsanlage 19 zum Beispiel mit einer Behandlungszone 4 derart leitungsverbunden sind, dass zumindest ein filtrierter und/oder bestrahlter Strom 46, 48, 49 der Prozessflüssigkeit der Behandlungszone 4 unter Einwirkung der Schwerkraft, in freiem Gefälle zugeführt werden kann. Ein solches Ausführungsbeispiel ist in Fig. 6 exemplarisch für die Zuführung eines filtrierten und bestrahlten Stromes 49 in eine Behandlungszone 4 dargestellt. Es werden in der Fig. 6 wiederum für gleiche Teile gleiche Bezugszeichen bzw. Bauteilbezeichnungen wie in den vorangegangenen Fig. 1 bis 5 verwendet werden, um unnötige Wiederholungen zu vermeiden.

In Fig. 6 ist ein Ausführungsbeispiel für eine leitungstechnische Verbindung einer UV-Bestrahlungseinrichtung 47 mit einer Behandlungszone 4 dargestellt, bei welcher ein von der UV-Bestrahlungseinrichtung 47 zur Behandlungszone 4 führendes Ableitungselement 24 derart angeordnet ist, dass ein stetiges Gefälle von oben nach unten in Richtung von der UV-Bestrahlungseinrichtung 47 zu der Behandlungszone 4 ausgebildet ist, wodurch der von der UV-Bestrahlungseinrichtung 47 zur Behandlungszone 4 geführte, bestrahlte und filtrierte Strom 49 der Prozessflüssigkeit 3 unter Schwerkrafteinwirkung fließen kann. Zur Einbringung des bestrahlten und filtrierten Stromes 49 der Prozessflüssigkeit 3 in die Behandlungszone 4 kann bzw. können in einfacher Art und Weise eine oder mehrere Öffnung(en) 25 in der Behandlungszone 4 ausgestaltet, bzw. mit den Ableitungselementen 24 leitungsverbunden sein, sodass der bestrahlte und filtrierte Strom 49 in die Behandlungszone 4 fließen kann.

Alternativ zu der in Fig. 6 dargestellten Ausführungsform kann an dieser Stelle in der Vorrichtung 1 statt der strömungstechnisch aufeinander folgenden Kombination aus einer Membranfiltrationsanlage 19 und einer UV-Bestrahlungseinrichtung 47 auch lediglich eine Membranfiltrationsanlage oder lediglich eine UV-Bestrahlungseinrichtung vorgesehen sein. Daher ist es ebenso möglich, dass ein lediglich unmittelbar filtrierter Strom oder ein lediglich unmittelbar bestrahlter Strom wenigstens einer Behandlungszone 4 zugeführt wird. Hierzu wären Ableitungselemente einer Membranfiltrationsanlage oder Ableitungselemente einer UV-Bestrahlungseinrichtung mit wenigstens einer Behandlungszone strömungstechnisch leitungsverbunden.

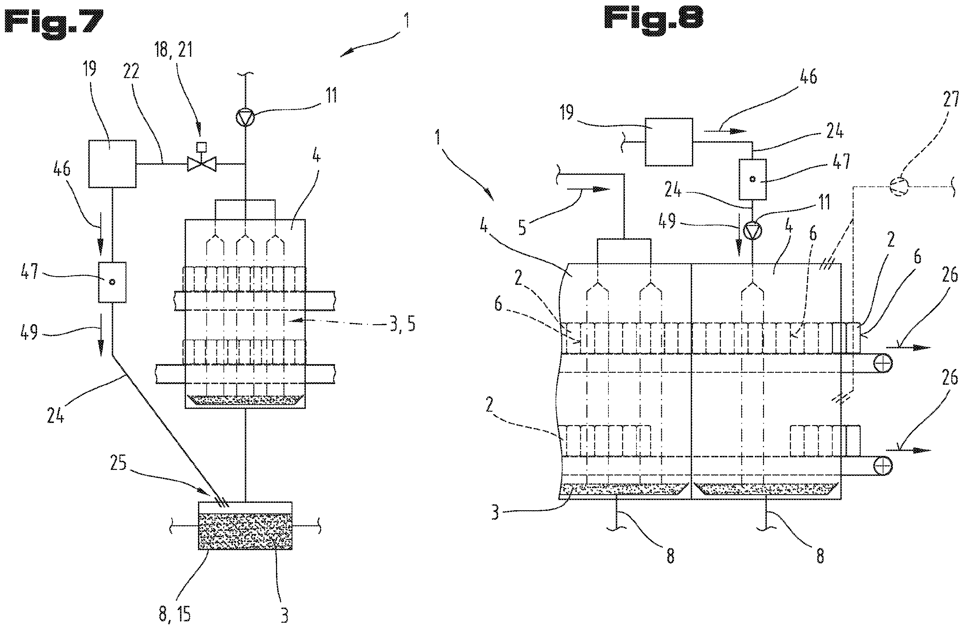

In Fig. 7 ist eine weitere und gegebenenfalls für sich eigenständige Ausführungsform der Vorrichtung 1 ausschnittsweise gezeigt, wobei wiederum für gleiche Teile gleiche Bezugszeichen bzw. Bauteilbezeichnungen, wie in den vorangegangenen Fig. 1 bis 6 verwendet werden. Um unnötige Wiederholungen zu vermeiden, wird auf die detaillierte Beschreibung in den vorangegangenen Fig. 1 bis 6 hingewiesen bzw. Bezug genommen. In Fig. 7 ist eine Anordnung zur Zuführung eines bestrahlten und filtrierten Stromes 49 der Prozessflüssigkeit 3 in ein Führungselement 8 für die Prozessflüssigkeit 3, zum Beispiel einen Flüssigkeitstank 15 dargestellt. Die Ableitungselemente 24 erstrecken sich wiederum in stetigem Gefälle von oben nach unten in Richtung von der UV-Bestrahlungseinrichtung 47 zum Flüssigkeitstank 8, 15, sodass der bestrahlte und filtrierte Strom 49 durch die Öffnung(en) 25 in den Flüssigkeitstank 8, 15 fließen kann.

Alternativ zu der in Fig. 7 dargestellten Ausführungsform kann an dieser Stelle wiederum in der Vorrichtung 1 statt der strömungstechnisch aufeinander folgenden Kombination aus einer Membranfiltrationsanlage 19 und einer UV-Bestrahlungseinrichtung 47 auch lediglich eine Membranfiltrationsanlage oder lediglich eine UV-Bestrahlungseinrichtung vorgesehen sein. Derart ist es wiederum möglich, dass ein lediglich unmittelbar filtrierter Strom oder ein lediglich unmittelbar bestrahlter Strom wenigstens einem Führungselement 8 zugeführt wird. Hierzu wären Ableitungselemente einer Membranfiltrationsanlage oder Ableitungselemente einer UV-Bestrahlungseinrichtung mit einem Führungselement strömungstechnisch leitungsverbunden.

In Fig. 8 ist eine weitere und gegebenenfalls für sich eigenständige Ausführungsform der Vorrichtung 1 ausschnittsweise gezeigt, wobei wiederum für gleiche Teile gleiche Bezugszeichen bzw. Bauteilbezeichnungen, wie in den vorangegangenen Fig. 1 bis 7 verwendet werden. Um unnötige Wiederholungen zu vermeiden, wird auf die detaillierte Beschreibung in den vorangegangenen Fig. 1 bis 7 hingewiesen bzw. Bezug genommen. In Fig. 8 ist eine Behandlungszone 4 zur Spülung der Außenseite 6 von mit dem Lebensmittel befüllten und verschlossenen Behältnissen 2 ausgestaltet, welche zumindest eine Behandlungszone 4 bezogen auf die Transportrichtung 26 der Behältnisse 2 durch die Behandlungszonen 4, am Ende der Behandlungszonenstrecke angeordnet ist. Im Ausführungsbeispiel gemäß Fig. 8 wird dieser Behandlungszone 4 zur Reinigung der Behältnisse 2 ein bestrahlter und filtrierter Strom 49 der Prozessflüssigkeit 3 zugeführt. Die Behandlungszone 4 ist zu diesem Zweck wiederum mit einem Ableitungselement 24 einer UV-Bestrahlungseinrichtung 47 strömungstechnisch leitungsverbunden. Weiters kann der Behandlungszone 4 beispielsweise ein Gebläse 27 zur Trocknung der Behältnisse 2 mittels Trocknungsluft zugeordnet, oder eine andere Trocknungsvorrichtung vorgesehen sein.

Auch zu der in Fig. 8 dargestellten Ausführungsform kann alternativ an dieser Stelle wiederum in der Vorrichtung 1 statt der strömungstechnisch aufeinander folgenden Kombination aus einer Membranfiltrationsanlage 19 und einer UV-Bestrahlungseinrichtung 47 auch lediglich eine Membranfiltrationsanlage oder lediglich eine UV-Bestrahlungseinrichtung vorgesehen sein. Auf diese Weise ist es möglich, dass ein lediglich unmittelbar filtrierter Strom oder ein lediglich unmittelbar bestrahlter Strom der Behandlungszone 4 zur Spülung der Behältnisse 2 zugeführt wird. Hierzu wären Ableitungselemente einer Membranfiltrationsanlage oder Ableitungselemente einer UV-Bestrahlungseinrichtung mit wenigstens einer Behandlungszone und/oder einem Führungselement strömungstechnisch leitungsverbunden.

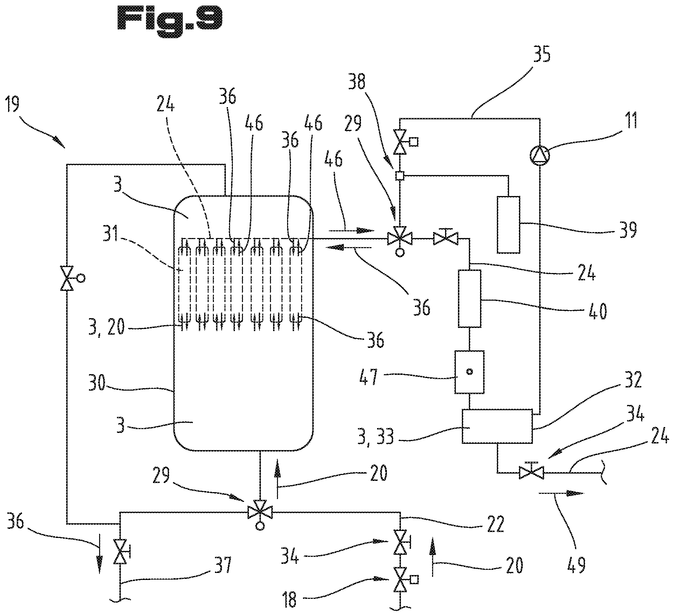

In Fig. 9 ist ein Ausführungsbeispiel für eine Ausgestaltungsform einer Membranfiltrationsanlage 19 dargestellt. Um unnötige Wiederholungen zu vermeiden, wird wiederum auf die detaillierte Beschreibung in den vorangegangenen Fig. 1 bis 8 hingewiesen bzw. Bezug genommen, bzw. werden für gleiche Teile gleiche Bezugszeichen bzw. Bauteilbezeichnungen, wie in den vorangegangenen Fig. 1 bis 8 verwendet. An dieser Stelle sei vermerkt, dass das in Fig. 9 gezeigte Ausführungsbeispiel für eine Membranfiltrationsanlage lediglich beispielhaft ist, und grundsätzlich auch anders ausgeführte Ausführungsformen einer Membranfiltrationsanlage für das Verfahren bzw. die Vorrichtung zur Behandlung von Lebensmitteln bzw. Behältnissen geeignet sein können.

Der Membranfiltrationsanlage 19 gemäß dem Ausführungsbeispiel in Fig. 9 wird im Filtrationsbetrieb, wie bereits ausführlich beschrieben, ein gebildeter Strom 20 der Prozessflüssigkeit 3 durch die Zuleitungselemente 22 zugeführt, wobei eine pro Zeiteinheit zugeführte Teilmenge zum Beispiel mittels einer Durchflussregeleinrichtung 18 festlegbar ist. Der zu filtrierende Strom 20 der Prozessflüssigkeit 3 kann beispielsweise über ein Drei-Wege-Ventil 29 in einen Druckbehälter 30 eingeleitet werden, in welchem Filtermembranmodule 31 zur Filtration der Prozessflüssigkeit 3 angeordnet sind.

Die in Fig. 9 dargestellten Filtermembranmodule 31 können Membranen verschiedenster Art umfassen. Die Membranen können homogen oder inhomogen in ihrem Aufbau sein und im Querschnitt verschiedene Symmetrien aufweisen. Insbesondere können poröse Membranen in Kapillar- bzw. Hohlfaserausführung und/oder Flachmembranen verwendet werden. Die Membranen können dabei aus diversen Materialien aufgebaut sein. Beispiele für geeignete Membranmaterialien sind Polyethylen, Polypropylen, Polyethersulfon, Polyvenylidenfluorid, Ethylen-Propylen-Dien-Kautschuk (EPDM), Polyurethan oder Zelluloseacetat. Bevorzugt werden Membranmaterialien in hydrophiler Ausbildung verwendet. Alternativ und/oder zusätzlich zu Kunststoffmembranen können auch keramische Materialien zur Bildung der Membranen der Filtermembranmodule 31 verwendet werden. Insbesondere sind chlorbeständige Membranmaterialien geeignet, welche gegenüber einer Chlorbelastung von mehr als 200.000 ppm*h, und bevorzugt mehr als 2.000.000 ppm*h dauerbeständig sind.

Das gezeigte Ausführungsbeispiel gemäß Fig. 9 zeigt einen Betrieb der Vorrichtung 1 bzw. der Membranfiltrationsanlage(n) 19 im Überdruckbetrieb. Alternativ bzw. zusätzlich können in der Vorrichtung 1 zumindest abschnittsweise auch Unterdruckzonen ausgebildet sein, insbesondere ist ein Unterdruckbetrieb einer Membranfiltrationsanlage 19 denkbar. Dabei können beispielsweise in den Ableitungselementen 24 Saugvorrichtungen (nicht dargestellt) angeordnet sein, mittels welcher ein filtrierter Strom 46 der Prozessflüssigkeit 3 aus einem Filtermembranmodul 31 abgezogen werden kann. Deshalb sind die Filtermembranen der Filtermembranmodule 31 bevorzugt gegenüber Überdruck und Unterdruck beständig ausgeführt und für transmembrane Drücke bzw. Druckdifferenzen von mindestens 1.000 mbar geeignet, ohne dass es zu einer permanenten Verblockung der Membran im Dauerbetrieb der Membranfiltrationsanlage 19 kommt. Je nach Bedarf können auch Membranen eingesetzt werden, welche für Drücke von beispielsweise 2.000 mbar und bis zu 5.000 mbar über die jeweilige Membran geeignet sind. Im Filtrationsbetrieb beträgt die transmembrane Druckdifferenz bevorzugt weniger als 5 bar, insbesondere weniger als 2 bar, und besonders bevorzugt 1 bar oder weniger. Bevorzugt werden poröse Membranen verwendet, wobei der wirksame Porendurchmesser einer jeweiligen Membran in einem Bereich zwischen 0,01 m und 1 m liegen kann, insbesondere sind Membranen mit wirksamen Porendurchmessern zwischen 0,05 m und 0,5 m für die Filtermembranmodule 31 der Membranfiltrationsanlage(n) 19 geeignet.

Im in Fig. 9 gezeigten Ausführungsbeispiel ist ein sogenannter 'Outside-In'-Betrieb der Membranfiltrationsanlage 19 dargestellt, bei welchem der zu filtrierende Strom 20 der Prozessflüssigkeit 3 im Filtrationsbetrieb von außen in die Filtermembranmodule 31 eintritt, über die Filtermembranen der Filtermembranmodule 31 filtriert, und ein filtrierter Strom 46 der Prozessflüssigkeit 3 mittels Ableitungselementen 24 aus dem Inneren der Filtermembranmodule 31 abgeleitet wird. Alternativ zum in Fig. 9 gezeigten Ausführungsbeispiel ist auch ein sogenannter 'Inside-Out'-Betrieb möglich, bei welchem ein zu filtrierender Strom 20 der Prozessflüssigkeit 3 im Filtrationsbetrieb ins Innere der Filtermembranmodule 31 geführt wird und ein filtrierter Strom 46 der Prozessflüssigkeit 3 an der Außenseite der Filtermembranmodule 31 austritt. Weiters sind betreffend den Fluss des Stromes 20 der Prozessflüssigkeit 3 in einem Filtermembranmodule 31 sowohl ein sogenannter 'cross-flow'-Betrieb als auch eine zyklische 'dead-end'-Verschaltung möglich. Schließlich sind auch getauchte Membrankonfigurationen, bei welchen ein filtrierter Strom 46 der Prozessflüssigkeit 3 mittels Unterdruck abgesaugt wird, möglich. Insbesondere bei einer Ausführung einer Membranfiltrationsanlage 19 in getauchter Konfiguration, kann zum Entgegenwirken der Deckschichtbildung auf den Membranoberflächen, auch eine zyklische oder a-zyklische Luftblasenspülung bzw. Luftverwirbelung vorgesehen sein, bzw. durchgeführt werden.

Im in Fig. 9 gezeigten Ausführungsbeispiel wird der filtrierte Strom 46 der Prozessflüssigkeit 3 nach Durchtritt der Prozessflüssigkeit 3 durch die Filtermembranmodule 31 und erfolgter Filtration über die Ableitungselemente 24 wieder aus der Membranfiltrationsanlage 19 abgeführt. Dabei kann es, wie in Fig. 9 gezeigt, zweckmäßig sein, in der Ableitung 24 einen Vorlagebehälter 32 mit Überlauf anzuordnen, welcher in Abhängigkeit seiner Dimensionen zur Zwischenspeicherung eines gewissen Volumens der gefilterten Prozessflüssigkeit 3 bzw. eines Filtrats 33 ausgestaltet ist. Insbesondere kann dieses Filtrat 33 der Prozessflüssigkeit 3 zur Reinigung durch Rückspülen, unter Umkehr der Flussrichtung über die Filtermembranmodule 31, genutzt werden.

Zur Durchführung eines Reinigungsbetriebs für die Filtermembranmodule 31 sind in den Zuleitungselementen 22 und den Ableitungselementen 24 Absperrmittel 34 angeordnet, welche ein strömungstechnisches Abtrennen der Membranfiltrationsanlage 19 von den weiteren baulichen Elementen der Vorrichtung zur Behandlung von Lebensmitteln bzw. Behältnissen erlauben. Weiters ist in dem Vorlagebehälter 32 und/oder einer sich zwischen dem Vorlagebehälter 32 und dem Ableitungselement 24 der Membranfiltrationsanlage 19 erstreckenden Rückspülleitung 35 zumindest ein Fördermittel 11 angeordnet. So kann durch entsprechendes Umschalten der Drei-Wege-Ventile 29 die Flussrichtung in der Membranfiltrationsanlage 19 umgekehrt werden, sodass die Prozessflüssigkeit 3 in zum Filtrationsbetrieb umgekehrter Richtung 36 über die Filtermembranmodule 31 strömt. Zum Abführen des im Zuge der Reinigung unter Umkehrung der Flussrichtung über die Filtermembranen der Membranfilteranlage 19 anfallenden Flüssigkeitsabfalls ist der Membranfiltrationsanlage 19 zumindest eine absperrbare Flüssigkeitsabfallleitung 37 zugeordnet. Eine, der abgeführten Menge an Flüssigkeitsabfall entsprechende Menge, an frischer Prozessflüssigkeit 3 kann zum Beispiel durch die in Fig. 2 dargestellte Zufuhrvorrichtung 17 für frische Prozessflüssigkeit 3 erfolgen.

Wie in Fig. 9 weiters dargestellt, kann in einem Ableitungselement 24 bzw. in der Rückspülleitung 35 der einer Membranfiltrationsanlage 19 eine Dosiereinrichtung 38 angeordnet sein, mittels welcher der Prozessflüssigkeit 3 bzw. dem Filtrat 33 der Prozessflüssigkeit 3 sowohl im Filtrationsbetrieb, als auch im Reinigungsbetrieb für die Membranfiltrationsanlage 19 Chemikalien aus einer oder mehreren Chemikalienquellen 39 beigemengt werden können. Ein Beimengen von Chemikalien kann im Filtrationsbetrieb über das in der Ableitung 24 angeordnete Drei-Wege-Ventil 29 erfolgen. In der Ableitung 24 der Membranfiltrationsanlage 19 kann weiters eine Adsorptionsvorrichtung 40 angeordnet sein, mittels welcher ein Entfernen bzw. Abscheiden von in einem filtrierten Strom 46 der Prozessflüssigkeit 3 gelöster bzw. suspendierter oder dispergierter Substanzen möglich ist.

Im Zusammenhang mit einer Reinigung der Filtermembranen einer Membranfiltrationsanlage 19 durch Rückspülung, kann es außerdem zweckmäßig sein, eine UV-Bestrahlungseinrichtung 47 zwischen einer Membranfiltrationsanlage 19 und einem Vorlagebehälter 32 anzuordnen, wie dies auch in der Fig. 9 beispielhaft dargestellt ist. Dadurch ist eine UV-Bestrahlung der im Vorlagebehälter 32 gesammelten Prozessflüssigkeit 3 während eines Rückspül- bzw. Reinigungsvorgangs für eine Membranfiltrationsanlage 19 ermöglicht, und kann zur Rückspülung der Filtermembranen eine Rückspülflüssigkeit mit besonders geringem Keimgehalt verwendet werden.

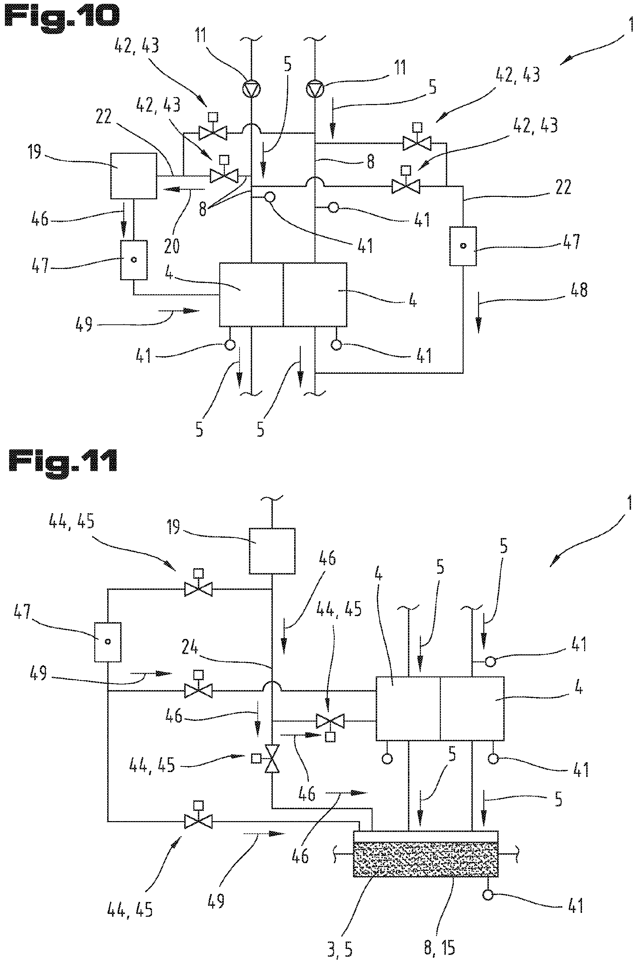

In Fig. 10 ist eine weitere und gegebenenfalls für sich eigenständige Ausführungsform der Vorrichtung 1 ausschnittsweise gezeigt, wobei wiederum für gleiche Teile gleiche Bezugszeichen bzw. Bauteilbezeichnungen, wie in den vorangegangenen Fig. 1 bis 9 verwendet werden. Um unnötige Wiederholungen zu vermeiden, wird auf die detaillierte Beschreibung in den vorangegangenen Fig. 1 bis 9 hingewiesen bzw. Bezug genommen. In Fig. 10 sind Sensoren 41 dargestellt, welche zur kontinuierlichen Überwachung des Verunreinigungsgrades, insbesondere durch Messen der Trübung der Prozessflüssigkeit, ausgestaltet sind. Sensoren 41 zur Messung bzw. Überwachung der Trübung der Prozessflüssigkeit können beispielsweise in den Führungselementen 8 und/oder in den Behandlungszonen 4 der Vorrichtung 1 angeordnet sein.

Die Messwerte der Sensoren 41 können herangezogen werden, um eine UV-Bestrahlungseinrichtung 47 und/oder eine Membranfiltrationsanlage 19 mittels Schaltelementen und Leitungselementen verschiedenen Behandlungszonen 4 bzw. Führungselementen 8 für Flüssigkeitsströme der Prozessflüssigkeit zuzuordnen, wie dies in der Fig. 10 exemplarisch dargestellt ist. Selbstverständlich kann ein Umschalten zwischen Führungselementen 8 und/oder Behandlungszonen 4 dabei auch in Abhängigkeit von Messungen anhand aus der Vorrichtung 1 entnommener Stichproben erfolgen.

Zum Zwecke eines Umschaltens einer Membranfiltrationsanlage 19 oder UV-Bestrahlungseinrichtung 47 auf verschiedene Führungselemente 8 können den Zuleitungselementen 22 einer UV-Bestrahlungseinrichtung 47 und/oder einer Membranfiltrationsanlage 19 zum Beispiel je zwei Umschaltmittel 42, 42 zugeordnet sein, wie dies in Fig. 10 veranschaulicht ist. Die beiden jeweils dargestellten Umschaltmittel 42, 42 sind dabei mit zwei verschiedenen, die Prozessflüssigkeit beinhaltenden, Führungselementen 8, 8 strömungstechnisch derart leitungsverbunden, dass eine Bildung eines Stromes 20 der Prozessflüssigkeit wahlweise aus einem der beiden Flüssigkeitsströme 5, 5 der Prozessflüssigkeit in den Führungselementen 8, 8, oder aus beiden Flüssigkeitsströmen 5, 5 erfolgen kann. Zu diesem Zweck können die jeweiligen beiden Umschaltmittel 42, 42 als sogenannte ,Auf/Zu-Ventile' ausgebildet sein, sodass jedes der beiden Umschaltmittel 42, 42 die Zufuhr von Prozessflüssigkeit in eine UV-Bestrahlungseinrichtung 47 und/oder eine Membranfiltrationsanlage 19 strömungstechnisch öffnen oder sperren kann. In dem in Fig. 10 dargestellten Ausführungsbeispiel ist der auf der linken Seite dargestellten Membranfiltrationsanlage 19 zusätzlich eine UV-Bestrahlungseinrichtung 47 strömungstechnisch unmittelbar nachgeschaltet.

Alternativ zum in Fig. 10 gezeigten Ausführungsbeispiel wären natürlich auch je ein, als 3-Wege-Umschaltmittel (in Fig. 10 nicht dargestellt) ausgebildetes, Umschaltmittel 42, zum Umschalten der Zuleitungselemente 22 der rechts dargestellten UV-Bestrahlungseinrichtung 47 und/oder der links dargestellten Membranfiltrationsanlage 19 auf eines der beiden Führungselemente 8 geeignet. Die als 3-Wege-Umschaltmittel 42 ausgebildeten Umschaltmittel 42 wäre wiederum einerseits den Zuleitungselementen 22 der UV-Bestrahlungseinrichtung 47 bzw. jenen der Membranfiltrationsanlage 19 zugeordnet, und andererseits mit zwei verschiedenen Führungselementen 8, 8 leitungsverbunden.

In Fig. 10 und im Folgenden wird zum besseren Verständnis die Darstellung und Beschreibung mittels 2-Wege-Umschalzmittel beibehalten, wobei an dieser Stelle angemerkt wird, dass die Anordnung einer ein- oder ausgangsseitig verschaltbaren UV-Bestrahlungseinrichtung 47 und/oder Membranfiltrationsanlage 19 auf zahlreiche Arten gebildet werden kann und nicht auf die in Fig. 10 dargestellten und im Folgenden dargestellten Ausführungsbeispiele beschränkt ist.

Anstelle von Umschaltmitteln 42 können einem Zuleitungselement 22 einer UV-Bestrahlungseinrichtung 47 und/oder einer Membranfiltrationsanlage 19 auch zwei Mischmittel 43, 43 zugeordnet sein, wie es in Fig. 10 angedeutet ist. Die Mischmittel 43, 43 sind wiederum mit zwei verschiedenen, die Prozessflüssigkeit beinhaltenden, Führungselementen 8, 8 strömungstechnisch derart leitungsverbunden, dass eine Bildung des Stromes 20 der Prozessflüssigkeit wiederum jeweils wahlweise aus einem der beiden Flüssigkeitsströme 5, 5 der Prozessflüssigkeit, oder aus beiden Flüssigkeitsströmen 5, 5 in den Führungselementen 8, 8, oder durch Entnahme und Mischung festlegbarer Teilmengen aus den beiden Flüssigkeitsströmen 5, 5 der Prozessflüssigkeit erfolgen kann. Zu diesem Zweck können die Mischmittel 43, 43 beispielsweise als Durchflussregelventile ausgestaltet sein.

Selbstverständlich können einer UV-Bestrahlungseinrichtung 47und/oder einer Membranfiltrationsanlage 19 auch mehr als zwei Umschaltmittel 42 und/oder Mischmittel 43 zugeordnet sein, welche dementsprechend mit mehr als zwei Führungselementen 8 leitungsverbunden sein können. Die Messwerte der Trübungs-Mess-Sensoren 41 können beispielsweise dazu verwendet werden, um eine Prozessflüssigkeit mit verhältnismäßig geringer Trübung direkt einer UV-Bestrahlungseinrichtung 47 zuzuführen. Andererseits kann auf Basis der Trübungsüberwachung auch eine verhältnismäßig stark verschmutzte bzw. trübe Prozessflüssigkeit einer Membranfiltrationsanlage 19 zugeführt werden.

In Fig. 11 ist eine weitere und gegebenenfalls für sich eigenständige Ausführungsform der Vorrichtung 1 ausschnittsweise gezeigt, wobei wiederum für gleiche Teile gleiche Bezugszeichen bzw. Bauteilbezeichnungen, wie in den vorangegangenen Fig. 1 bis 10 verwendet werden. Um unnötige Wiederholungen zu vermeiden, wird auf die detaillierte Beschreibung in den vorangegangenen Fig. 1 bis 10 hingewiesen bzw. Bezug genommen. In dem in Fig. 11 dargestellten Ausführungsbeispiel sind einer Ableitung 24 einer Membranfiltrationsanlage 19 drei Umschaltelemente 44, 44 zugeordnet. Ein Umschaltelement 44 ist mit einem als Flüssigkeitstank 15 ausgebildeten Führungselement 8 strömungstechnisch leitungsverbunden. Ein weiteres Umschaltelement 44 ist mit einer Behandlungszone 4 strömungstechnisch leitungsverbunden. Ein drittes Umschaltelement 44 ist mit einer UV- Bestrahlungseinrichtung 47 leitungsverbunden. Durch diese, beispielhafte Ausgestaltungsform der Vorrichtung 1 kann eine Zuführung eines filtrierten Stromes 46 der Prozessflüssigkeit 3 wahlweise in das als Flüssigkeitstank 15 ausgebildete Führungselement 8 oder die Behandlungszone 4 oder die UV-Bestrahlungseinrichtung 47, oder sowohl in das Führungselement 8 die Behandlungszone 4, als auch in die UV-Bestrahlungseinrichtung 47 erfolgen.

Zu diesem Zweck können die drei Umschaltelemente 44, 44, 44 in Fig. 11 wiederum als sogenannte "Auf/Zu-Ventile" ausgebildet sein, sodass jedes der drei Umschaltelemente 44, 44, 44 die Abführung eines filtrierten Stromes 46 aus der Membranfiltrationsanlage 19 in die Behandlungszone 4 und/oder in das wenigstens eine Führungselement 8 und/oder die zumindest eine UV-Bestrahlungseinrichtung 47 strömungstechnisch öffnen oder sperren kann.

Anstelle von Umschaltelementen 44 können einem Ableitungselement 24 einer Membranfiltrationsanlage 19 auch drei Verteilerelemente 45, 45, 45 zugeordnet sein, wie es in Fig. 11 angedeutet ist. Ein Verteilerelement 45 ist wiederum mit einem als Flüssigkeitstank 15 ausgebildeten Führungselement 8 strömungstechnisch leitungsverbunden. Ein zweites Verteilerelement 45 ist mit einer Behandlungszone 4 strömungstechnisch leitungsverbunden. Ein drittes Verteilerelement 45 ist schließlich mit der dargestellten UV-Bestrahlungseinrichtung 47 leitungsverbunden. Durch diese Ausgestaltungsform kann eine Zuführung des filtrierten Stromes 46 der Prozessflüssigkeit 3 wieder wahlweise in das als Flüssigkeitstank 15 ausgebildete Führungselement 8 und/oder die Behandlungszone 4 und/oder die UV-Bestrahlungseinrichtung 47 erfolgen. Alternativ können dem Flüssigkeitstank 15 und/oder der Behandlungszone 4 und/oder der UV-Bestrahlungseinrichtung 47 jeweils festlegbare Teilmengen des filtrierten Stromes 46 der Prozessflüssigkeit 3 zugeführt werden. Zu diesem Zweck können die Verteilerelemente 45, 45, 45 beispielsweise wiederum als Durchflussregelventile ausgestaltet sein.Wiederum können einer Membranfiltrationsanlage 19 auch mehr als drei Umschaltelemente 44 und/oder Verteilerelemente 45 zugeordnet sein, welche dementsprechend mit mehreren Führungselementen 8 und/oder mehreren Behandlungszonen 4 und/oder mehreren UV-Bestrahlungseinrichtungen 47 leitungsverbunden sein können.

Die in Fig. 11 dargestellte UV-Bestrahlungseinrichtung 47 ist ausgangsseitig über Umschaltelemente 44 und/oder Verteilerelemente 45 mit zumindest einer Behandlungszone 4 und/oder mit zumindest einem Führungselement 8, 15 leitungsverbunden, um einen filtrierten und bestrahlten Strom 49 der Behandlungszone 4 und/oder dem Führungselement 8 zuführen zu können.

Die Ausführungsbeispiele zeigen mögliche Ausführungsvarianten des Verfahrens bzw. der Vorrichtung zur Behandlung von Lebensmitteln und/oder Behältnissen, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Der Erfindungsgegenstand wird durch die Ansprüche definiert.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10, mit umfasst sind, d.h. sämtliche Teilbereiche beginnen mit einer unteren Grenze von 1 oder größer und enden bei einer oberen Grenze von 10 oder weniger, z.B. 1 bis 1,7 oder 3,2 bis 8,1 oder 5,5 bis 10.

Der Ordnung halber sei abschließend darauf hingewiesen, dass zum besseren Verständnis des Aufbaus der Vorrichtung zur Behandlung von Lebensmitteln und/oder Behältnissen diese bzw. deren Bestandteile teilweise unmaßstäblich und/oder vergrößert und/oder verkleinert dargestellt wurden.

**Bezugszeichenaufstellung**

| | | | |
|---|---|---|---|
| 1 | Vorrichtung | 31 | Filtermembranmodul |
| 2 | Behältnis | 32 | Vorlagebehälter |
| 3 | Prozessflüssigkeit | 33 | Filtrat |
| 4 | Behandlungszone | 34 | Absperrmittel |
| 5 | Flüssigkeitsstrom | 35 | Rückspülleitung |
| 6 | Außenseite | 36 | Richtung |
| 7 | Sprühdüse | 37 | Flüssigkeitsabfallleitung |
| 8 | Führungselement | 38 | Dosiereinrichtung |
| 9 | Sammelwanne | 39 | Chemikalienquelle |
| 10 | Transportmittel | 40 | Adsorptionsvorrichtung |
| 11 | Fördermittel | 41 | Sensor |
| 12 | Heizmittel | 42 | Umschaltmittel |
| 13 | Kühlmittel | 43 | Mischmittel |
| 14 | Flüssigkeitstank | 44 | Umschaltelement |
| 15 | Flüssigkeitstank | 45 | Verteilerelement |
| 16 | Entleerungsvorrichtung | 46 | Strom |
| 17 | Zufuhrvorrichtung | 47 | UV-Bestrahlungseinrichtung |
| 18 | Durchflussregeleinrichtung | 48 | Strom |
| 19 | Membranfiltrationsanlage | 49 | Strom |
| 20 | Strom | 50 | Durchflussbehälter |
| 21 | Verteilungsmittel | | |
| 22 | Zuleitungselement | | |
| 23 | Drei-Wege-Verteilerventil | | |
| 24 | Ableitungselement | | |
| 25 | Öffnung | | |
| 26 | Transportrichtung | | |
| 27 | Gebläse | | |
| 28 | Innenseite | | |
| 29 | Drei-Wege-Ventil | | |
| 30 | Druckbehälter | | |

## Patentansprüche

1. Verfahren zur Behandlung von Lebensmitteln in zumindest einer Behandlungszone (4), wobei die zu behandelnden Lebensmittel vor der Behandlung in Behältnisse (2) abgefüllt werden, die Behältnisse (2) verschlossen werden, und die Behältnisse (2) in eine Behandlungszone (4) eingebracht und/oder durch eine Behandlungszone (4) befördert werden,
und wobei der Behandlungszone (4) oder den Behandlungszonen (4) jeweils zumindest ein Flüssigkeitsstrom (5) einer Prozessflüssigkeit (3) zum Einwirken auf die Behältnisse (2) zugeführt wird, wobei ein jeweiliger Flüssigkeitsstrom (5) der Prozessflüssigkeit (3) vor Zuführung in eine Behandlungszone (4) temperiert wird, und die Behandlung der Lebensmittel in einer Behandlungszone (4) durch Wärmeübertragung mittels einer temperierten Prozessflüssigkeit (3) durchgeführt wird, indem die Prozessflüssigkeit (3) eine Außenseite (6) der Behältnisse (2) umströmt, und wobei die Prozessflüssigkeit (3) nach erfolgter Behandlung der Lebensmittel aus der oder den Behandlungszone(n) (4) wieder abgeführt wird,
wobei die Temperaturen der jeweiligen Flüssigkeitsströme (5) der Prozessflüssigkeit (3) vor der Zuführung in eine Behandlungszone (4) separat für jede Behandlungszone (4) eingestellt werden, und die Lebensmittel in zumindest einer Behandlungszone (4) pasteurisiert werden, und wobei die zu behandelnden Lebensmittel aufeinanderfolgend in zumindest einer Behandlungszone (4) erwärmt werden, in zumindest einer Behandlungszone (4) pasteurisiert werden und in zumindest einer Behandlungszone (4) abgekühlt werden, und wobei ein Flüssigkeitsstrom (5) der Prozessflüssigkeit (3) wenigstens einer Behandlungszone (4) zum Erwärmen der Lebensmittel und/oder Behältnisse (2) bei einer Temperatur zwischen 40°C und 50°C zugeführt wird,
und wobei die Prozessflüssigkeit (3) zur Behandlung der Lebensmittel zwecks Wiederverwendung im Verfahren im Kreis wieder in die Behandlungszone (4) oder in die Behandlungszonen (4) geführt wird,
**dadurch gekennzeichnet, dass**
aus der pro Zeiteinheit insgesamt im Kreis durch alle vorhandenen Behandlungszonen (4) geführten Prozessflüssigkeit (3) pro Zeiteinheit eine Teilmenge der Prozessflüssigkeit (3) zur Bildung wenigstens eines Stroms (20) der Prozessflüssigkeit (3) herangezogen wird,
und der wenigstens eine gebildete Strom (20) der Prozessflüssigkeit (3) mittels zumindest einer Membranfiltrationsanlage (19) filtriert wird, und/oder mittels zumindest einer UV-Bestrahlungseinrichtung (47) bestrahlt wird,
und ein bestrahlter Strom und/oder filtrierter Strom (46, 48, 49) der Prozessflüssigkeit (3) in zumindest ein die Prozessflüssigkeit beinhaltendes und/oder führendes Führungselement (8) oder in zumindest eine Behandlungszone (4) zurückgeführt wird,
wobei mittels zumindest eines verstellbaren Verteilungsmittels (21) oder mehrerer zusammenwirkender Verteilungsmittel (21) pro Zeiteinheit eine festlegbare Menge der Prozessflüssigkeit (3) aus zumindest einem die Prozessflüssigkeit (3) beinhaltenden bzw. führenden Element (8) entnommen wird und zur Bildung des wenigstens einen Stromes (20) der Prozessflüssigkeit (3) herangezogen wird,
und wobei die im kontinuierlichen Behandlungsbetrieb aus zumindest einem die Prozessflüssigkeit beinhaltenden und/oder führenden Führungselement (8) pro Zeiteinheit insgesamt zur Bildung wenigstens eines Stromes (20) der Prozessflüssigkeit (3) herangezogene Prozessflüssigkeitsmenge so gewählt wird, dass durch die Bestrahlung und/oder Filtration des Stromes (20) oder der Ströme (20) eine Entfernungsrate für Mikroorganismen erzielbar ist, welche größer ist, als die Wachstumsrate der Mikroorganismen in der Prozessflüssigkeit (3) in der gleichen Zeiteinheit,
und wobei zur Bildung des wenigstens einen zu filtrierenden Stromes (20) Prozessflüssigkeit (3) mit einer Temperatur zwischen 40°C und 50°C herangezogen wird.

2. Verfahren nach Anspruch 1, **dadurch gekennzeichnet, dass** wenigstens ein filtrierter Strom (46) der Prozessflüssigkeit (3) unmittelbar nachfolgend auf einen Filtrationsvorgang einer UV-Bestrahlungseinrichtung (47) zugeführt und bestrahlt wird, und ein filtrierter und bestrahlter Strom (49) der Prozessflüssigkeit (3) zumindest einem die Prozessflüssigkeit beinhaltenden und/oder führenden Führungselement (8) und/oder zumindest einer Behandlungszone (4) wieder zugeführt wird.

3. Verfahren nach Anspruch 1, **dadurch gekennzeichnet, dass** der wenigstens eine Strom (20) durch Entnahme der Prozessflüssigkeit (3) aus einem temperierbaren Durchflussbehälter (50) für die Prozessflüssigkeit (3) gebildet wird.

4. Verfahren nach einem oder mehreren der vorangehenden Ansprüche, **dadurch gekennzeichnet, dass** ein bestrahlter und/oder filtrierter Strom (46, 48, 49) der Prozessflüssigkeit (3) bei Umgebungsdruck in freiem Gefälle in zumindest ein die Prozessflüssigkeit beinhaltendes und/oder führendes Führungselement (8) und/oder in zumindest eine Behandlungszone (4) zurückgeführt wird.

5. Verfahren nach einem oder mehreren der vorangehenden Ansprüche, **dadurch gekennzeichnet, dass** ein bestrahlter und/oder filtrierter Strom (46, 48, 49) der Prozessflüssigkeit (3) zumindest teilweise einer im Verfahren zur Behandlung von in verschlossenen Behältnissen (2) abgefüllten Lebensmitteln am Ende des Prozesses angeordneten Behandlungszone (4) zur Spülung der Außenseite (6) von mit Lebensmitteln befüllten und verschlossenen Behältnissen (2) zugeführt wird.

6. Verfahren nach einem oder mehreren der vorangehenden Ansprüche, **dadurch gekennzeichnet, dass** der Verunreinigungsgrad der Prozessflüssigkeit (3) insbesondere durch Messen der Trübung der Prozessflüssigkeit mittels in Führungselementen (8) und/oder in Behandlungszonen (4) angeordneten Sensoren (41) kontinuierlich überwacht wird.

7. Verfahren nach einem oder mehreren der vorangehenden Ansprüche, **dadurch gekennzeichnet, dass** ein Strom (20) der Prozessflüssigkeit (3) bedarfsabhängig aus unterschiedlichen, die Prozessflüssigkeit beinhaltenden und/oder führenden Führungselementen (8) gebildet wird, mittels zumindest einer Membranfiltrationsanlage (19) filtriert wird, und ein filtrierter Strom (46) nach dem Membranfiltrationsvorgang zumindest einem die Prozessflüssigkeit beinhaltenden und/oder führenden Führungselement (8) und/oder zumindest einer Behandlungszone (4) und/oder zumindest einer UV-Bestrahlungseinrichtung (47) zugeführt wird.

8. Vorrichtung (1) für die Behandlung von in verschlossenen Behältnissen (2) befindlichen Lebensmitteln mittels einer Prozessflüssigkeit (3), umfassend zumindest eine Behandlungszone (4), welche Behandlungszone (4) zur Applikation der Prozessflüssigkeit (3) auf eine Außenseite (6) von verschlossenen Behältnissen (2) ausgestaltet ist, wobei die Prozessflüssigkeit (3) die Außenseite (6) der verschlossenen Behältnisse (2) umströmt,
Transportmittel (10) zum Transport der Behältnisse (2) durch die Behandlungszone(n) (4), sowie die Prozessflüssigkeit beinhaltende und/oder führende Führungselemente (8) zum Zuführen von Flüssigkeitsströmen (5) der Prozessflüssigkeit (3) in eine Behandlungszone (4) und Führungselemente (8) zur Abführung von Flüssigkeitsströmen (5) der Prozessflüssigkeit (3) aus der oder den Behandlungszone(n) (4),
weitere Führungselemente (8) zur Beinhaltung und/oder Führung der Prozessflüssigkeit (3) in der Vorrichtung (1) und wenigstens ein Fördermittel (11) zur Förderung von Flüssigkeitsströmen (5) der Prozessflüssigkeit (3) in den Führungselementen (8), wobei die Führungselemente (8) derart ausgestaltet und angeordnet sind, dass die Prozessflüssigkeit (3) im Kreis wieder in die Behandlungszone (4) oder in die Behandlungszonen (4) geführt werden kann,
wobei die Vorrichtung (1) zumindest ein Heizmittel (12) zur Erwärmung der Prozessflüssigkeit (3) und zumindest ein Kühlmittel (13) zur Abkühlung der Prozessflüssigkeit (3) umfasst,
**dadurch gekennzeichnet, dass**
die Vorrichtung (1) zumindest eine UV-Bestrahlungseinrichtung (47) und zumindest eine Membranfiltrationsanlage (19) umfasst, wobei die zumindest eine UV-Bestrahlungseinrichtung (47) und die zumindest eine Membranfiltrationsanlage (19) derart mit den Führungselementen (8) und/oder mit den Behandlungszonen (4) strömungstechnisch leitungsverbunden sind, dass eine Teilmenge der pro Zeiteinheit insgesamt im Kreis durch alle vorhandenen Behandlungszonen (4) geführten Prozessflüssigkeit (3) zur Bildung wenigstens eines Stromes (20) der Prozessflüssigkeit herangezogen, der gebildete Strom (20) oder die gebildeten Ströme (20) mittels der zumindest einen Membranfiltrationsanlage (19) filtriert und/oder mittels der zumindest einen UV-Bestrahlungseinrichtung (47) bestrahlt, und ein filtrierter und/oder bestrahlter Strom (46, 48, 49) der Prozessflüssigkeit zumindest einem Führungselement (8) oder zumindest einer Behandlungszone (4) zugeführt werden kann,
wobei die zumindest eine Membranfiltrationsanlage (19) Bypass-artig zwischen einem einen Flüssigkeitsstrom (5) führenden Führungselement (8) und einer Behandlungszone (4) oder einem Führungselement (8) angeordnet ist, und wobei die zumindest eine UV-Bestrahlungseinrichtung (47) Bypass-artig zwischen zwei Führungselementen (8) oder zwischen einem Führungselement (8) und einer Behandlungszone (4) angeordnet ist,
und wobei eingangsseitig der zumindest einen UV-Bestrahlungseinrichtung (47) und/oder der zumindest einen Membranfiltrationsanlage (19) zumindest ein verstellbares Verteilungsmittel (21) oder mehrere, zusammenwirkende Verteilungsmittel (21) zur Entnahme einer festlegbaren Prozessflüssigkeitsmenge pro Zeiteinheit aus zumindest einem die Prozessflüssigkeit (3) beinhaltenden bzw. führenden Führungselement (8), und zur Bildung des wenigstens einen zu bestrahlenden und/oder zu filtrierenden Stromes (20) der Prozessflüssigkeit (3) angeordnet sind.

9. Vorrichtung nach Anspruch 8, **dadurch gekennzeichnet, dass** aufeinanderfolgend entlang einer Transportrichtung (26) der Lebensmittel oder Behältnisse (2) zumindest eine Behandlungszone (4) zur Erwärmung der Lebensmittel und/oder Behältnisse (2), zumindest eine Behandlungszone (4) zur Pasteurisierung der Lebensmittel, und zumindest eine Behandlungszone (4) zur Abkühlung der Lebensmittel und/oder Behältnisse (2) vorgesehen sind.

10. Vorrichtung nach Anspruch 8 oder 9, **dadurch gekennzeichnet, dass** wenigstens eine UV-Bestrahlungseinrichtung (47) strömungstechnisch unmittelbar nachfolgend auf eine Membranfiltrationsanlage (19) angeordnet ist, sodass ein filtrierter Strom (46) der Prozessflüssigkeit unmittelbar nach erfolgter Filtration mittels der UV-Bestrahlungseinrichtung (47) bestrahlt werden kann.

11. Vorrichtung nach einem oder mehreren der Ansprüche 8 bis 10, **dadurch gekennzeichnet, dass** ein Zuleitungselement (22) einer Membranfiltrationsanlage (19) mit einem temperierbaren Durchflussbehälter (50) für die Prozessflüssigkeit (3) leitungsverbunden ist.

12. Vorrichtung nach einem oder mehreren der Ansprüche 8 bis 11, **dadurch gekennzeichnet, dass** die Anzahl und Bestrahlungsleistung der UV-Bestrahlungseinrichtung(en) (47) sowie die Anzahl und die Filtrationskapazität der Membranfiltrationsanlage(n) (19) derart festgelegt sind, dass die im kontinuierlichen Behandlungsbetrieb aus zumindest einem die Prozessflüssigkeit beinhaltenden und/oder führenden Führungselement (8) pro Zeiteinheit insgesamt zur Bildung wenigstens eines Stromes (20) der Prozessflüssigkeit herangezogene Prozessflüssigkeitsmenge so gewählt werden kann, dass durch die Filtration und Bestrahlung des Stromes (20) oder der Ströme (20) eine Entfernungsrate für Mikroorganismen erzielbar ist, welche größer ist als die Wachstumsrate der Mikroorganismen in der Prozessflüssigkeit in derselben Zeiteinheit.

13. Vorrichtung nach einem oder mehreren der Ansprüche 8 bis 12, **dadurch gekennzeichnet, dass** zur Rückführung eines bestrahlten und/oder filtrierten Stromes (46, 48, 49) der Prozessflüssigkeit Ableitungselemente (24) von zumindest einer UV-Bestrahlungseinrichtung (47) und/oder zumindest einer Membranfiltrationsanlage (19) mit zumindest einem Führungselement (8) und/oder zumindest einer Behandlungszone (4) derart leitungsverbunden sind, dass ein bestrahlter und/oder filtrierter Strom (46, 48, 49) der Prozessflüssigkeit dem oder den Führungselement(en) (8) und/oder der oder den Behandlungszone(n) (4) unter Einwirkung von Schwerkraft in freiem Gefälle zugeführt werden kann.

14. Vorrichtung nach einem oder mehreren der Ansprüche 8 bis 13, **dadurch gekennzeichnet, dass** zumindest eine Behandlungszone (4) zur Spülung der Außenseite (6) von mit Lebensmitteln befüllten und verschlossenen Behältnissen (2) ausgestaltet ist, welche bezogen auf eine Transportrichtung (26) der Behältnisse (2) durch die Behandlungszonen (4) am Ende der Behandlungszonenstrecke angeordnet ist und welche Behandlungszone (4) zur Spülung der Behältnisse durch Zuführung eines bestrahlten und/oder filtrierten Stromes (46, 48, 49) der Prozessflüssigkeit mit zumindest einem Ableitungselement (24) einer UV-Bestrahlungseinrichtung (47) und/oder einem Ableitungselement (24) einer Membranfiltrationsanlage (19) leitungsverbunden ist.

15. Vorrichtung nach einem oder mehreren der Ansprüche 8 bis 14, **dadurch gekennzeichnet, dass** in Führungselementen (8) und/oder in Behandlungszonen (4) Sensoren (41) zur kontinuierlichen Überwachung des Verunreinigungsgrades der Prozessflüssigkeit (3), insbesondere durch Messen der Trübung der Prozessflüssigkeit (3), angeordnet sind.

16. Vorrichtung nach einem oder mehreren der Ansprüche 8 bis 15, **dadurch gekennzeichnet, dass** einem Zuleitungselement (22) einer Membranfiltrationsanlage (19) zumindest ein Umschaltmittel (42) zugeordnet ist, welches mit wenigstens zwei verschiedenen, die Prozessflüssigkeit beinhaltenden, Führungselementen (8) strömungstechnisch derart leitungsverbunden ist, dass eine Bildung des zu filtrierenden Stromes (20) der Prozessflüssigkeit bedarfsabhängig aus einem der Flüssigkeitsströme (5) oder mehreren Flüssigkeitsströmen (5) der Prozessflüssigkeit (3) in den Führungselementen (8), oder aus mehreren Flüssigkeitsströmen (5) erfolgen kann.

17. Vorrichtung nach einem oder mehreren der Ansprüche 8 bis 16, **dadurch gekennzeichnet, dass** einem Zuleitungselement (22) einer Membranfiltrationsanlage (19) zumindest ein Mischmittel (43) zugeordnet ist, welches mit wenigstens zwei verschiedenen die Prozessflüssigkeit beinhaltenden Führungselementen (8) strömungstechnisch derart leitungsverbunden ist, dass eine Bildung des zu filtrierenden Stromes (20) der Prozessflüssigkeit wahlweise aus einem der Flüssigkeitsströme (5) oder mehreren Flüssigkeitsströmen (5) der Prozessflüssigkeit in den Führungselementen (8) erfolgen kann oder die Bildung eines zu filtrierenden Stromes (20) der Prozessflüssigkeit durch die Membranfiltrationsanlage (19) durch Entnahme und Mischung festlegbarer Teilmengen aus mehreren Flüssigkeitsströmen (5) der Prozessflüssigkeit erfolgen kann.

## Claims

1. A method for treating foods in at least one treatment zone (4), wherein the foods to be treated are filled into containers (2) before the treatment, the containers (2) are closed, and the containers (2) are introduced into a treatment zone (4) and/or conveyed through a treatment zone (4),
and wherein the treatment zone (4) or the treatment zones (4) are each fed at least one liquid stream (5) of a process liquid (3) to act on the containers (2), wherein a particular liquid stream (5) of the process liquid (3) is tempered before being fed into a treatment zone (4), and the treatment of the foods is executed in a treatment zone (4) by heat transfer by means of a tempered process liquid (3), by the process liquid (3) flowing around an outside (6) of the containers (2), and wherein the process liquid (3) is drained again after completed treatment of the foods out of the treatment zone(s) (4),
wherein the temperatures of the particular liquid streams (5) of the process liquid (3) are set separately for each treatment zone (4) before feeding into a treatment zone (4), and the foods are pasteurized in at least one treatment zone (4), and wherein the foods to be treated are heated successively in at least one treatment zone (4), are pasteurized in at least one treatment zone (4), and are cooled in at least one treatment zone (4), and wherein a liquid stream (5) of the process liquid (3) is fed into at least one treatment zone (4) for heating the foods and/or containers (2) at a temperature of between 40°C and 50°C,
and wherein the process liquid (3) is recirculated into the treatment zone (4) or the treatment zones (4) for re-use in the method,
**characterized in that**
out of the total process liquid (3) conducted through all existing treatment zones (4) per time unit, a partial quantity of the process liquid (3) is used per time unit to form at least one stream (20) of the process liquid (3),
and the at least one formed stream (20) of the process liquid (3) is filtered by means of at least one membrane filtration system (19) and/or irradiated by means of at least one UV irradiation apparatus (47),
an irradiated stream and/or filtered stream (46, 48, 49) of the process liquid (3) is fed back into at least one conduction element (8) containing and/or conducting the process liquid and/or into at least one treatment zone (4),
wherein a specifiable quantity of the process liquid (3) is removed out of at least one element (8) containing and/or conducting the process liquid (3) per time unit by means of at least one adjustable splitting means (21) or multiple cooperating splitting means (21) and is used for the formation of the at least one stream (20) of the process liquid (3),
and wherein the process liquid quantity used to form at least one stream (20) of the process liquid (3) out of at least one conduction element (8) containing and/or conducting the process liquid during continuous treatment operation per time unit is selected in such a way that the irradiation and/or filtration of the stream (20) or the streams (20) allow(s) for a removal rate for micro-organisms to be achieved that is larger than the growth rate of the micro-organisms in the process liquid (3) in the same time unit,
and wherein process liquid (3) with a temperature of between 40°C and 50°C is used to form the at least one stream (20) to be filtered.

2. The method according to claim 1, **characterized in that** at least one filtered stream (46) of the process liquid (3) is fed into a UV irradiation apparatus (47) and irradiated immediately after the filtration process, and a filtered and irradiated stream (49) of the process liquid (3) is fed back into at least one conduction element (8) containing and/or conducting the process liquid and/or at least one treatment zone (4).

3. The method according to claim 1, **characterized in that** the at least one stream (20) is formed by removing the process liquid (3) from a temperature-controllable flow-through container (50) for the process liquid (3).

4. The method according to one or more of the preceding claims, **characterized in that** an irradiated and/or filtered stream (46, 48, 49) of the process liquid (3) is fed back into at least one conduction element (8) containing and/or conducting the process liquid and/or into at least one treatment zone (4) at ambient pressure in free fall.

5. The method according to one or more of the preceding claims, **characterized in that** an irradiated and/or filtered stream (46, 48, 49) of the process liquid (3) is at least partially fed into a treatment zone (4) arranged at the end of the process in the method for treating foods filled into closed containers (2) for rinsing the outside (6) of closed containers filled with foods (2).

6. The method according to one or more of the preceding claims, **characterized in that** the degree of contamination of the process liquid (3) is continuously monitored, in particular by measuring the turbidity of the process liquid by means of sensors (41) arranged in conduction elements (8) and/or in treatment zones (4).

7. The method according to one of the preceding claims, **characterized in that** a stream (20) of the process liquid (3) is formed as needed out of different conduction elements (8) containing and/or conducting the process liquid, is filtered by means of at least one membrane filtration system (19), and after the membrane filtration process a filtered stream (46) is fed into at least one conduction element (8) containing and/or conducting the process liquid and/or at least one treatment zone (4) and/or at least one UV irradiation apparatus (47).

8. A device (1) for treating foods in closed containers (2) by means of a process liquid (3), comprising at least one treatment zone (4), which treatment zone (4) is designed to apply the process liquid (3) to the outside (6) of closed containers (2), wherein the process liquid (3) flows around the outside (6) of the closed containers (2),
means of transport (10) for transporting the containers (2) through the treatment zone(s) (4) and conduction elements (8) containing and/or conducting the process liquid for feeding liquid streams (5) of the process liquid (3) into a treatment zone (4) and conduction elements (8) for discharging liquid streams (5) of the process liquid (3) from the treatment zone(s) (4),
further conduction elements (8) for containing and/or conducting the process liquid (3) in the device (1) and at least one conveying means (11) for conveying liquid streams (5) of the process liquid (3) in the conduction elements (8), wherein the conduction elements (8) are designed and arranged such that the process liquid (3) can be recirculated again into the treatment zone (4) or into the treatment zones (4),
wherein the device (1) comprises at least one heating means (12) for heating the process liquid (3) and at least one cooling means (13) for cooling the process liquid (3),
**characterized in that**
the device (1) comprises at least one UV irradiation apparatus (47) and at least one membrane filtration system (19), wherein the at least one UV irradiation apparatus (47) and the at least one membrane filtration system (19) are fluidically line-connected to the conduction elements (8) and/or to the treatment zones (4) in such a way that a partial amount of the total process liquid (3) fed through all existing treatment zones (4) per time unit can be used to form at least one stream (20) of the process liquid, the formed stream (20) or the formed streams (20) can be filtered by means of the at least one membrane filtration system (19) and/or irradiated by means of the at least one UV irradiation apparatus (47), and a filtered and/or irradiated stream (46, 48, 49) of the process liquid can be fed into at least one conduction element (8) or at least one treatment zone (4),
wherein the at least one membrane filtration system (19) is arranged between a conduction element (8) conducting a liquid stream (5) and a treatment zone (4) or a conduction element (8) in bypass-like manner, and wherein the at least one UV irradiation apparatus (47) is arranged between two conduction elements (8) or between a conduction element (8) and a treatment zone (4) in bypass-like manner,
wherein at least one adjustable splitting means (21) or multiple cooperating splitting means (21) are arranged on an inlet side of the at least one UV irradiation apparatus (47) and/or the at least one membrane filtration system (19) for removing a specifiable process liquid quantity per time unit out of at least one conduction element (8) containing and/or conducting the process liquid (3), and for the formation of the at least one stream (20) of the process liquid (3) to be irradiated and/or filtered.

9. The device according to claim 8, **characterized in that** at least one treatment zone (4) for heating the foods and/or containers (2), at least one treatment zone (4) for pasteurizing the foods, and at least one treatment zone (4) for cooling the foods and/or containers (2) are provided in succession along the transport direction (26) of the foods or containers (2).

10. The device according to claim 8 or 9, **characterized in that** at least one UV irradiation apparatus (47) is fluidically arranged immediately successive to a membrane filtration system (19) such that a filtered stream (46) of the process liquid can be irradiated by means of the UV irradiation apparatus (47) immediately after performed filtration.

11. The device according to one or more of claims 8 to 10, **characterized in that** a feeding element (22) of a membrane filtration system (19) is line-connected to a temperature-controllable flow-through container (50) for the process liquid (3).

12. The device according to one or more of claims 8 to 11, **characterized in that** the number and irradiation power of the UV irradiation apparatus(es) (47) and the number and filtration capacity of the membrane filtration system(s) (19) are fixed such that the total process liquid drawn out of at least one conduction element (8) containing and/or conducting the process liquid per time unit for forming at least one stream (20) of the process liquid during continuous treatment operation can be selected such that the filtration and irradiation of the stream (20) or the streams (20) allow for a removal rate of micro-organisms to be achieved that is greater than the growth rate of the micro-organisms in the process liquid in the same time unit.

13. The device according to one or more of claims 8 to 12, **characterized in that** in order to recirculate an irradiated and/or filtered stream (46, 48, 49) of the process liquid, draining elements (24) of at least one UV irradiation apparatus (47) and/or of at least one membrane filtration system (19) are line-connected to at least one conduction element (8) and/or at least one treatment zone (4) in such a way that an irradiated and/or filtered stream (46, 48, 49) of the process liquid can be fed into the conduction element(s) (8) and/or the treatment zone(s) (4) under the influence of gravity in free fall.

14. The device according to one or more of claims 8 to 13, **characterized in that** at least one treatment zone (4) for rinsing the outside (6) of closed containers (2) filled with foods is provided, which is arranged at the end of the treatment zone line in the transport direction (26) of the containers (2) through the treatment zones (4) and which treatment zone (4) is line-connected to at least one draining element (24) of a UV irradiation apparatus (47) and/or a draining element (24) of a membrane filtration system (19) in order to rinse the containers by feeding in an irradiated and/or filtered stream (46, 48, 49) of the process liquid.

15. The device according to one or more of claims 8 to 14, **characterized in that** sensors (41) are arranged in conduction elements (8) and/or in treatment zones (4) to continuously monitor the degree of contamination of the process liquid (3), in particular by measuring the turbidity of the process liquid (3).

16. The device according to one or more of claims 8 to 15, **characterized in that** at least one switching means (42) is assigned to a feeding element (22) of a membrane filtration system (19) that is fluidically line-connected to at least two different conduction elements (8) containing the process liquid in such a way that the stream (20) of process liquid to be filtered can be formed as needed either from one of the liquid streams (5) or multiple liquid streams (5) of the process liquid (3) in the conduction elements (8) or from multiple liquid streams (5).

17. The device according to one or more of claims 8 to 16, **characterized in that** at least one mixing means (43) is assigned to a feeding element (22) of a membrane filtration system (19) that is fluidically line-connected to at least two different conduction elements (8) containing the process liquid in such a way that the stream (20) of process liquid to be filtered can be formed as desired either from one of the liquid streams (5) or multiple liquid streams (5) of the process liquid in the conduction elements (8) or a stream (20) of the process liquid to be filtered by the membrane filtration system (19) can be formed by removing and mixing specifiable partial quantities from multiple liquid streams (5) of the process liquid.

## Revendications

1. Procédé de traitement de produits alimentaires dans au moins une zone de traitement (4), dans lequel les produits alimentaires à traiter sont placé dans des récipients (2) avant le traitement, les récipients (2) sont fermés, et les récipients (2) sont introduits dans une zone de traitement (4) et/ou transportés à travers une zone de traitement (4),
et dans lequel respectivement au moins un flux de liquide (5) d'un liquide de process (3) est acheminé à la zone de traitement (4) ou aux zones de traitement (4) pour agir sur les récipients (2), dans lequel un flux de liquide (5) respectif du liquide de process (3) est tempéré avant l'acheminement dans une zone de traitement (4), et le traitement des produits alimentaires dans une zone de traitement (4) est réalisé par transmission de chaleur au moyen d'un liquide de process (3) tempéré par le fait que le liquide de process (3) s'écoule autour d'un côté extérieur (6) des récipients (2), et dans lequel le liquide de process (3), une fois effectué le traitement des produits alimentaires, est évacué de nouveau de la ou des zones de traitement (4),
les températures des flux de liquide (5) respectifs du liquide de process (3) sont réglées séparément pour chaque zone de traitement (4) avant l'acheminement dans une zone de traitement (4), et les produits alimentaires sont pasteurisés dans au moins une zone de traitement (4), et dans lequel les produits alimentaires à traiter sont chauffés les uns après les autres dans au moins une zone de traitement (4), sont pasteurisés dans au moins une zone de traitement (4) et sont refroidis dans au moins une zone de traitement (4), et dans lequel un flux de liquide (5) du liquide de process (3) est acheminé au moins à une zone de traitement (4) pour le chauffage des produits alimentaires et/ou des récipients (2) à une température entre 40 °C et 50 °C,
et dans lequel, pour le traitement des produits alimentaires à des fin de réutilisation en boucle dans le procédé, le liquide de process (3) est conduit de nouveau dans la zone de traitement (4) ou dans les zones de traitement (4),
**caractérisé en ce que**
à partir du liquide de process (3) conduit par unité de temps au total en boucle à travers toutes les zones de traitement (4) existantes, une quantité partielle du liquide de process (3) est utilisée par unité de temps pour la formation d'au moins un flux (20) du liquide de process (3),
et le au moins un flux (20) formé du liquide de process (3) est filtré au moyen d'au moins une installation de filtration par membrane (19) et/ou est irradié au moyen d'au moins un équipement d'irradiation UV (47),
et un flux irradié et/ou flux filtré (46, 48, 49) du liquide de process (3) est renvoyé dans au moins un élément (8) contenant et/ou conduisant le liquide de process ou dans au moins une zone de traitement (4),
dans lequel, au moyen d'au moins un moyen de répartition (21) réglable ou de plusieurs moyens de répartition (21) qui coopèrent, une quantité pouvant être fixée du liquide de process (3) est prélevée par unité de temps à partir du au moins un élément (8) contenant ou respectivement conduisant le liquide de process (3) et est utilisée pour la formation d'au moins un flux (20) du liquide de process (3),
et dans lequel la quantité de liquide de process utilisée dans le mode de traitement continu à partir d'au moins un élément (8) contenant et/ou conduisant le liquide de process par unité de temps au total pour la formation d'au moins un flux (20) du liquide de process (3) est choisie de telle sorte que, par l'irradiation et/ou la filtration du flux (20) ou des flux (20), il est possible d'atteindre un taux d'enlèvement pour des microorganismes qui est supérieur au taux de croissance des microorganismes dans le liquide de process (3) dans le même intervalle de temps,
et dans lequel du liquide de process (3) à une température entre 40 °C et 50 °C est utilisé pour la formation du au moins un flux (20) à filtrer.

2. Procédé selon la revendication 1, **caractérisé en ce qu'**au moins un flux filtré (46) du liquide de process (3) est acheminé à un équipement d'irradiation UV (47) et irradié directement à la suite d'un processus de filtration, et un flux filtré et irradié (49) du liquide de process (3) est acheminé de nouveau au moins à un élément (8) contenant et/ou conduisant le liquide de process (3) et/ou au moins à une zone de traitement (4).

3. Procédé selon la revendication 1, **caractérisé en ce que** le au moins un flux (20) est formé par le prélèvement du liquide de process (3) à partir d'un réservoir de circulation (50), pouvant être tempéré, pour le liquide de process (3).

4. Procédé selon l'une ou plusieurs des revendications précédentes, **caractérisé en ce qu'**un flux irradié et/ou filtré (46, 48, 49) du liquide de process (3) est, à la pression ambiante, par gravité, acheminé dans au moins un élément (8) contenant et/ou conduisant le liquide de process et/ou dans au moins une zone de traitement (4).

5. Procédé selon l'une ou plusieurs des revendications précédentes, **caractérisé en ce que**, pour le lavage du côté extérieur (6) de récipients (2) remplis de produits alimentaires et fermés, un flux irradié et/ou filtré (46, 48, 49) du liquide de process (3) est acheminé au moins partiellement à une zone de traitement (4) disposée à l'extrémité du processus dans le procédé de traitement de produits alimentaires mis en place dans des récipients (2) fermés.

6. Procédé selon l'une ou plusieurs des revendications précédentes, **caractérisé en ce que** le degré de contamination du liquide de process (3) est surveillé en continu en particulier par le mesurage de la turbidité du liquide de process au moyen de capteurs (41) disposés dans des éléments de guidage (8) et/ou dans des zones de traitement (4).

7. Procédé selon l'une ou plusieurs des revendications précédentes, **caractérisé en ce qu'**en fonction des besoins un flux (20) du liquide de process (3) est formé de différents éléments (8) contenant et/ou conduisant le liquide de process, est filtré au moyen d'au moins une installation de filtration par membrane (19), et un flux filtré (46) est, après le processus de filtration par membrane, acheminé au moins à un élément (8) contenant et/ou conduisant le liquide de process (3) et/ou au moins à une zone de traitement (4) et/ou au moins à un équipement d'irradiation UV (47).

8. Dispositif (1) de traitement de produits alimentaires situés dans des récipients (2) fermés au moyen d'un liquide de process (3), comprenant au moins une zone de traitement (4), laquelle zone de traitement (4) est constituée pour l'application du liquide de process (3) sur un côté extérieur (6) de récipients (2) fermés, dans lequel le liquide de process (3) s'écoule autour du côté extérieur (6) des récipients (2) fermés,
des moyens de transport (10) destinés au transport des récipients (2) à travers la / les zone(s) de traitement (4), ainsi que des éléments (8) contenant et/ou conduisant le liquide de process destinés à l'acheminement de flux de liquide (5) du liquide de process (3) dans une zone de traitement (4), et des éléments de guidage (8) destinés à l'évacuation de flux de liquide (5) du liquide de process (3) à partir de la ou des zone(s) de traitement (4),
d'autres éléments de guidage (8) destinés à contenir et/ou à guider le liquide de process (3) dans le dispositif (1) et au moins un moyen de transfert (11) destiné au transfert de flux de liquide (5) du liquide de process (3) dans les éléments de guidage (8), dans lequel les éléments de guidage (8) sont constitués et disposés de telle sorte que le liquide de process (3) peut être conduit de nouveau en boucle dans la zone de traitement (4) ou dans les zones de traitement (4),
dans lequel le dispositif (1) comprend au moins un moyen de chauffage (12) pour le chauffage du liquide de process (3) et au moins un moyen de refroidissement (13) pour le refroidissement du liquide de process (3),
**caractérisé en ce que**
le dispositif (1) comprend au moins un équipement d'irradiation UV (47) et au moins une installation de filtration par membrane (19), dans lequel le au moins un équipement d'irradiation UV (47) et la au moins une installation de filtration par membrane (19) sont raccordés fluidiquement par conduite aux éléments de guidage (8) et/ou aux zones de traitement (4) de telle sorte qu'une quantité partielle du liquide de process (3) conduit par unité de temps au total en boucle à travers toutes les zones de traitement (4) existantes peut être utilisée pour la formation d'au moins un flux (20) du liquide de process (3), le flux (20) formé ou les flux (20) formés peut / peuvent être filtré(s) au moyen de la au moins une installation de filtration par membrane (19) et/ou irradiés au moyen du au moins un équipement d'irradiation UV (47), et un flux irradié et/ou filtré (46, 48, 49) du liquide de process (3) peut être conduit au moins à un élément de guidage (8) ou au moins à une zone de traitement (4),
dans lequel la au moins une installation de filtration par membrane (19) est disposée en forme de dérivation entre un élément de guidage (8) guidant un flux de liquide (5) et une zone de traitement (4) ou un autre élément de guidage (8), et dans lequel le au moins un équipement d'irradiation UV (47) est disposé en forme de dérivation entre deux éléments de guidage (8) ou entre un élément de guidage (8) et une zone de traitement (4),
et dans lequel, côté entrée du au moins équipement d'irradiation UV (47) et/ou de la au moins une installation de filtration par membrane (19), au moins un moyen de répartition (21) réglable ou plusieurs moyens de répartition (21) qui coopèrent est / sont disposé(s) pour le prélèvement par unité de temps d'une quantité de liquide de process pouvant être fixée à partir d'au moins un élément de guidage (8) contenant ou respectivement guidant le liquide de process (3), et pour la formation du au moins un flux (20) à irradier et/ou à filtrer du liquide de process (3).

9. Dispositif selon la revendication 8, **caractérisé en ce que**, de façon successive le long d'une direction de transport (26) des produits alimentaires ou des récipients (2), il est prévu au moins une zone de traitement (4) destinée au chauffage des produits alimentaires et/ou des récipients (2), au moins une zone de traitement (4) destinée à la pasteurisation des produits alimentaires, et au moins une zone de traitement (4) destinée au refroidissement des produits alimentaires et/ou des récipients (2).

10. Dispositif selon la revendication 8 ou 9, **caractérisé en ce qu'**au moins un équipement d'irradiation UV (47) est disposé fluidiquement directement à la suite d'une installation de filtration par membrane (19) de telle sorte qu'un flux filtré (46) du liquide de process peut, tout de suite après la filtration, être irradié au moyen de l'équipement d'irradiation UV (47).

11. Dispositif selon l'une ou plusieurs des revendications 8 à 10, **caractérisé en ce qu'**un élément d'alimentation (22) d'une installation de filtration par membrane (19) est raccordé par conduite à un réservoir à circulation (50), pouvant être tempéré, pour le liquide de process (3).

12. Dispositif selon l'une ou plusieurs des revendications 8 à 11, **caractérisé en ce que** le nombre et la puissance d'irradiation de l'élément de l'équipement / des équipements d'irradiation UV (47) ainsi que le nombre et la capacité de filtration de la / des installation(s) de filtration par membrane (19) sont fixés de telle sorte que la quantité de liquide de process utilisée par unité de temps dans le mode de traitement continu à partir d'au moins un élément (8) contenant et/ou conduisant le liquide de process au total pour la formation d'au moins un flux (20) du liquide de process (3) peut être choisie de telle sorte que la filtration et l'irradiation du flux (20) ou des flux (20) permet d'obtenir un taux d'enlèvement pour des microorganismes qui est supérieur au taux de croissance des microorganismes dans le liquide de process dans la même unité de temps.

13. Dispositif selon l'une ou plusieurs des revendications 8 à 12, **caractérisé en ce que**, pour le renvoi d'un flux irradié et/ou filtré (46, 48, 49) du liquide de process, des éléments de dérivation (24) d'au moins un équipement d'irradiation UV (47) et/ou d'au moins une installation de filtration par membrane (19) sont raccordés par conduite à au moins un élément de guidage (8) et/ou à au moins une zone de traitement (4) de telle sorte qu'un flux irradié et/ou filtré (46, 48, 49) du liquide de process peut être acheminé à l'élément ou aux élément(s) de guidage (8) et/ou à la ou aux zone(s) de traitement (4) sous l'action de la pesanteur par gravité.

14. Dispositif selon l'une ou plusieurs des revendications 8 à 13, **caractérisé en ce qu'**au moins une zone de traitement (4) est constituée pour le lavage du côté extérieur (6) de récipients (2) remplis de produits alimentaires et fermés, laquelle est disposée à l'extrémité du parcours de la zone traitement par rapport à un sens de transport (26) des récipients (2) à travers les zones de traitement (4), et laquelle zone de traitement (4) est, pour le lavage des récipients par l'acheminement d'un flux irradié et/ou filtré (46, 48, 49) du liquide de process (3), raccordée par conduite à au moins un élément de dérivation (24) d'un équipement d'irradiation UV (47) et/ou à un élément de dérivation (24) d'une installation de filtration par membrane (19).

15. Dispositif selon l'une ou plusieurs des revendications 8 à 14, **caractérisé en ce que** des capteurs (41) destinés à la surveillance continue du degré de contamination du liquide de process (3), en particulier par le mesurage de la turbidité du liquide de process (3), sont disposés dans des éléments de guidage (8) et/ou dans des zones de traitement (4).

16. Dispositif selon l'une ou plusieurs des revendications 8 à 15, **caractérisé en ce qu'**au moins un moyen de commutation (42) est affecté à un élément d'alimentation (22) d'une installation de filtration par membrane (19), lequel est raccordé par conduite fluidiquement à au moins deux éléments de guidage (8) différents contenant le liquide de process de telle sorte qu'une formation du flux (20) à filtrer du liquide de process peut être effectuée en fonction des besoins à partir d'un des flux de liquide (5) ou de plusieurs flux de liquide (5) du liquide de process (3) dans les éléments de guidage (8), ou à partir de plusieurs flux de liquide (5).

17. Dispositif selon l'une ou plusieurs des revendications 8 à 16, **caractérisé en ce qu'**au moins un moyen de mixage (43) est affecté à un élément d'alimentation (22) d'une installation de filtration par membrane (19), lequel est raccordé par conduite fluidiquement à au moins deux éléments de guidage (8) différents contenant le liquide de process de telle sorte qu'une formation du flux (20) à filtrer du liquide de process peut être effectuée au choix à partir d'un des flux de liquide (5) ou de plusieurs flux de liquide (5) du liquide de process dans les éléments de guidage (8), ou la formation d'un flux (20) à filtrer du liquide de process à travers l'installation de filtration par membrane (19) peut être effectuée par prélèvement et mixage de quantités partielles pouvant être fixées à partir de plusieurs flux de liquide (5) du liquide de process.
